# EUROPEAN PATENT APPLICATION

(11) **EP 2 233 566 A1**
(43) Date of publication of application: **29.09.2010**
(21) Application number: 09155331.3
(22) Date of filing: 17.03.2009
(51) Int. Cl.: C12N 5/077

(54) **Generation of pancreatic progenitor cells**

(71) Applicant: Vrije Universiteit Brussel, 1050 Brussel (BE); OPUS NV, 1761 Borchtlombeek (BE)
(72) Inventor: Bouwens, Luc, 1653 Dworp (BE); Kunjom Mfopou, Josué, 1785 Merchtem (BE)
(74) Representative: Brants, Johan P.E.

(57) **Abstract**

The invention relates to *in vitro* methods for producing pancreatic progenitor cells and cell populations from cells having definitive endoderm germ layer phenotype. The invention further encompasses so-obtained pancreatic progenitor cells and cell populations, compositions comprising the same, and further uses of said pancreatic progenitor cells and cell populations, such as *inter alia* for producing functional, insulin-producing beta cells. The invention is of particular relevance for medical applications, more specifically in the field of cell therapy of diabetes.

## Description

### FIELD OF THE INVENTION

The invention relates to *in vitro* methods for producing pancreatic progenitor cells and cell populations from cells having definitive endoderm germ layer phenotype. The invention further encompasses so-obtained pancreatic progenitor cells and cell populations, compositions comprising the same, and further uses of said pancreatic progenitor cells and cell populations, such as *inter alia* for producing functional, insulin-producing beta cells. The invention is of particular relevance for medical applications, more specifically in the field of cell therapy of diabetes.

### BACKGROUND OF THE INVENTION

Precise regulation of circulating glucose levels, *i.e.,* physiologically adequate glucose homeostasis, is essential for proper functioning and health of organisms, and it is well-documented that disturbances of glucose homeostasis can characterise and/or contribute to the aetiology of several prevalent diseases. In particular, diabetes mellitus metabolically manifests in elevated blood glucose levels, commonly accompanied by further clinically relevant symptoms, and in long term by cardiovascular, neurological, ocular and/or renal complications.

Insulin plays a central role in glucose homeostasis and causes a reduction in the circulating glucose levels, generally by increasing the uptake, metabolism and/or storage of glucose in cells of peripheral tissues, most prominently the adipose and muscle tissues. Accordingly, useful therapeutic interventions in disorders of glucose homeostasis may target the production and/or levels of insulin.

Insulin is a polypeptide hormone synthesised and secreted by the beta (β) cells of the islets of Langerhans of the pancreas. In diabetes mellitus pancreatic beta cells may be destroyed by body's own immune defense mechanisms (*i.e*., Type 1, juvenile onset or insulin-dependent diabetes), or there may occur a relative decrease in beta cell mass and function (Type 2, adult onset or non insulin-dependent diabetes). Whereas the etiological mechanism of Type 2 diabetes differs from that of Type 1 at onset, there is in at least some cases and at a certain time point of disease progression a common landmark manifested in the decrease in beta cell mass.

Replacement therapy of pancreatic beta cells under the form of islet cell transplantation is currently one of the best options for treatment of Type 1 diabetes, and can also be used to treat late-stage Type 2 diabetes. For example, transplantation of isolated islet cells obtained from cadaveric donors allows for insulin-independence for periods as long as five years. However, the therapy is seriously hampered by the shortage of islet donor material. Consequently, there exists a continuing need to provide for alternative sources of islet- or beta-cell material.

Recently, others have suggested certain *in vitro* protocols to differentiate cells of definitive endoderm germ layer phenotype into pancreatic progenitor cells, in the widely acknowledged prediction that such cells can eventually provide a source of mature, insulin-producing beta cells.

An exemplary protocol was disclosed by D'Amour et al. 2006 (Nat Biotechnol 24: 1392-401). However, others (Cho et al. 2008. Biochemical and Biophysical Research Communications 366: 129-34; Semb et al. 2008. Biochem Soc Trans 36: 272-5) could not reproduce the original results of D'Amour *et al.* 2006. Moreover, when the present Applicant subjected four distinct human embryonic stem cell lines to the protocol of D'Amour *et al.* 2006, this generated hepatoblast-like cells, which subsequently differentiated to hepatocyte-like cells having functional characteristics of mature hepatocytes. This suggest that the original protocol of D'Amour *et al.* 2006 may intrinsically favour the generation of cells of hepatic lineage at the expense of the desired pancreatic cells.

Hence, despite the refinement of culture conditions, only limited amounts of beta-like cells could be previously obtained *in vitro* and cells of non-pancreatic lineages also developed in culture, though not usually characterised. This indicates that the success rate may be at least in part limited by too low proportion of pancreas progenitors established at relatively early to mid stages of such cultures.

In view hereof, there exists a need for alternative and/or improved *in vitro* differentiation methods, so as to increase the yield of pancreatic progenitors and pancreatic endocrine progenitors. Such progenitors can expectedly provide a valuable source of cells for differentiation towards pancreatic beta cells *in vitro* or *in vivo.*

### SUMMARY OF THE INVENTION

The present invention is at least in part based on the following main findings:
- Exposure of nascent human embryonic stem cells-derived definitive endoderm cells to ligands of the FGF family induced a population of alpha-fetoprotein-positive cells that further expressed low levels of albumin, representing a hepatoblast population;
- The above hepatoblast-like cells further lost alpha-fetoprotein and upregulated albumin, transthyretin, alpha1-antitrypsine and glucose-6-phoshatase. These cells closely matched mature hepatocytes as they also secreted albumin in the medium, stored glycogen and synthesized and released urea;
- A combination of bone morphogenetic protein (BMP) antagonist and fibroblast growth factor (FGF)-receptor inhibitor abolished the observed differentiation of hepatic cells, but was not associated with any significant improvement of pancreatic markers expression, notably Pdx1;
- Combined application of BMP antagonist, hedgehog antagonist and retinoic acid without supplemented FGF ligands and without suppressing the endogenous FGF signalling allowed for the establishment of early pancreas progenitors mainly characterized by Foxa2, Sox9 and Pdx1 expression;
- Exposure of said early pancreas progenitors to FGF signalling and Notch inhibitor induced a large proportion of Pdx1-positive cells that expressed other transcription factors characteristic of the pancreatic and endocrine progenitor including but not limited to Foxa2, Sox9, Hnf6, Nkx6.1 and Ngn3.

To better appreciate the relevance of some of the assessed markers, it is noted that during mammalian embryonic life, pancreas originates from two buds of the posterior foregut endoderm, a derivative of the definitive endoderm germ layer. The gut endoderm region which gives rise to the pancreas is initially marked by its expression of the transcription factor Pdx1. Later on, the Pdx1-expressing pancreas progenitors differentiate into exocrine, duct and endocrine cells. All the endocrine cells within islets (*i.e.,* including insulin-producing beta cells (∼70%), glucagon-producing alpha cells (∼20%), somatostatin-producing delta cells (∼5%), pancreatic polypeptide (PP)-producing cells (∼1%) and ghrelin-producing epsilon cells (∼1%)) originate from a common progenitor characterised by a transient expression of Ngn3.

Hence, the Applicant has *inter alia* confirmed that pancreas progenitors can be differentiated from definitive endoderm (such as, for example, from ES cell-derived definitive endoderm) and that the proportion of pancreas progenitors can be increased at the expense of liver cells by combining, preferably during the early stages of differentiation, blockade of hepatic lineage commitment (in particular, BMP antagonism) with the use of agents that inhibit hedgehog pathway and preferably also agents that activate retinoid signalling.

The methods disclosed herein thus advance the efforts to generate alternative and abundant sources of beta cells needed for allowing the application of cell therapy to the ever-growing number of diabetic patients.

Accordingly, in an aspect the invention provides an *in vitro* method for producing pancreatic progenitor cells or a cell population comprising such cells, comprising the steps of: (a) providing cells having definitive endoderm germ layer phenotype; and (b) exposing the cells of (a) to one or more agents or conditions that inhibit differentiation of said cells along hepatic lineage and to one or more agents or conditions that induce differentiation of said cells along pancreatic lineage. Hereby, the production of pancreas progenitors is enhanced at the expense of hepatic cells, in particular hepatoblasts.

Another aspect provides an *in vitro* method for producing cells that have early gut endoderm phenotype and that substantially cannot differentiate along hepatic lineage, or a cell population comprising such cells, comprising the steps of: (a) providing cells having definitive endoderm germ layer phenotype; and (b) exposing the cells of (a) to one or more agents or conditions that inhibit differentiation of said cells along hepatic lineage.

In an embodiment, the one or more agents or conditions that inhibit differentiation of the cells having definitive endoderm germ layer phenotype along hepatic lineage may be chosen from the group comprising or consisting of: an antagonist of BMP pathway, an antagonist of FGF signalling, and/or the absence of an exogenous agonist of FGF signalling.

In another embodiment, the one or more agents or conditions that induce differentiation of the cells having definitive endoderm germ layer phenotype along pancreatic lineage may be chosen from the group comprising or consisting of: an antagonist of hedgehog pathway and an agonist of retinoid signalling.

Hence, a preferred aspect of the invention provides an *in vitro* method for producing pancreatic progenitor cells or a cell population comprising such, comprising the steps of: (a) providing cells having definitive endoderm germ layer phenotype; and (b) exposing the cells of (a) to an antagonist of bone morphogenetic protein (BMP) pathway and to one or both of, preferably both of, an antagonist of hedgehog pathway and an agonist of retinoid signalling.

Hence, a preferred embodiment of the method comprises the steps of (a) providing cells having definitive endoderm germ layer phenotype; and (b) exposing the cells of (a) to an antagonist of BMP pathway, an antagonist of hedgehog pathway and an agonist of retinoid signalling.

In embodiments, the antagonist of BMP pathway may be chosen from the group comprising or consisting of noggin, chordin, grhemlin, sclerostin, Cerberus, GDF3 (growth/differentiation factor-3), Dan, Coco, PRDC, short gastrulation and twisted gastrulation. Preferably, the antagonist of BMP pathway may be noggin.

In embodiments, the antagonist of hedgehog pathway may be chosen from the group comprising or consisting of cyclopamine, KAAD-cyclopamine, tomatidine, jervine, cholesterol, tripanarol, AY9944, and anti-hedgehog antibodies. Preferably the antagonist of hedgehog pathway may be cyclopamine or KAAD-cyclopamine.

In embodiments, the agonist of retinoid signalling may be chosen from the group comprising or consisting of retinoic acid, retinol and retinal. Preferably, said agonist of retinoid signalling may be all-trans retinoic acid.

Preferably, the present methods may employ mammalian cells, more preferably human cells.

In an embodiment, the cells having definitive endoderm germ layer phenotype as provided in step (a) of the present methods may be derived or obtained from (e.g., differentiated from) pluripotent stem cells, preferably from mammalian pluripotent stem (mPS) cells, more preferably from human pluripotent stem (hPS) cells. Preferably, the cells as provided in step (a) may be obtainable or directly obtained by *in vitro* method comprising the steps of: (i) providing pluripotent stem cells, preferably mPS cells, more preferably hPS cells; and (ii) differentiating said pluripotent stem cells, mPS cells or hPS cells into cells having definitive endoderm germ layer phenotype.

It shall be understood that said step (a) may provide a suitable cell population comprising or enriched for said cells having definitive endoderm germ layer phenotype.

Cells having definitive endoderm germ layer phenotype as intended herein preferably comprise the expression of any one of, more preferably of any two or more of, even more preferably all of Bry (brachyury), Sox17 (SRY(sex determining region Y)-box 17), Gsc (goosecoid homeobox), Foxa2 (forkhead box A2 ) and CxCR4 (chemokine (C-X-C motif) receptor 4).

Preferably, said expression of Bry may be transient. By means of example, when the definitive endoderm germ layer cells are produced by differentiation from pluripotent stem cells, the cells may comprise the expression of Bry between day 1 and day 3 of said differentiation, more typically between day 1 and day 2 of said differentiation, even more typically on day 1 of said differentiation, whereby the expression of Bry substantially decreases thereafter (e.g., decreases by at least 5-fold, more typically by at least 10-fold, even more typically by at least 15-fold or more between day 1 and day 3 of said differentiation). Hence, once obtained or produced, cells having definitive endoderm germ layer phenotype as intended herein may preferably comprise the expression of any one of, more preferably any two or more of, even more preferably all of Sox17, Gsc, Foxa2 and CxCR4.

Also preferably, the cells having definitive endoderm germ layer phenotype as intended herein may comprise co-localisation of proteins Foxa2 and Sox17 and/or co-localisation of proteins Foxa2 and CxCR4. Further preferably, said cells having definitive endoderm germ layer phenotype as intended herein may be negative for Sox7 (SRY (sex determining region Y)-box 7) expression. Typically, the cells having definitive endoderm germ layer phenotype may display epithelial morphology.

In an embodiment, the cells in said step (b) of the present methods may be exposed to any two or more of the antagonist of BMP pathway and the antagonist of hedgehog pathway and/or the agonist of retinoid signalling simultaneously or sequentially in any order. Preferably, at least the antagonist of BMP pathway and the antagonist of hedgehog pathway may be included at the start of step (b). Preferably, the agonist of retinoid signalling may also be included at the outset of step (b). Otherwise, the agonist of retinoid signalling may be included in the course of step (b), such as, e.g., at about 1 day after the start of step (b), or at about 2 days after the start of step (b), or at about 3 days after the start of step (b), or at about 4 days after the start of step (b) (for example, it may be included starting at these time points for the rest of the duration of step (b)). Preferably, the antagonist of BMP pathway and the antagonist of hedgehog pathway may be included throughout the duration of step (b). In alternative, any one or both of the antagonist of BMP pathway and the antagonist of hedgehog pathway, and more particularly, the antagonist of BMP pathway, may be withdrawn in the course of step (b), such as, e.g., may be withdrawn at about 2 day after the start of step (b), or at about 3 days after the start of step (b), or at about 4 days after the start of step (b) (for example, may be withdrawn starting at these time points for the rest of the duration of step (b)). More preferably, the cells in said step (b) of the present methods may be exposed to the antagonist of BMP pathway and the antagonist of hedgehog pathway and/or the agonist of retinoid signalling, simultaneously, more preferably simultaneously and throughout the duration of said step (b).

In another embodiment, the cells in said step (b) of the present methods are not exposed to an exogenous agonist of FGF signalling.

In a further embodiment, the cells in said step (b) of the present methods may be exposed to an antagonist of FGF signalling. It may be preferred that the cells in said step (b) of the present methods are not exposed to an antagonist of FGF signalling.

The duration of step (b) may be generally chosen to be adequate to allow for the pancreatic progenitors to form. Said adequate duration may be determined by detecting the presence (*e.g*., by detecting the expression of corresponding markers) of pancreatic progenitor cells in the resulting cell population. By means of example, an adequate duration may be concluded when the resulting cell population comprises at least about 5%, at least about 10%, preferably at least about 15%, more preferably at least about 20% and even more preferably at least about 30% or more (*e.g*., at least about 40%, at least about 50%, at least about 60%, at least about 70%, at least about 80% or at least about 90%) of pancreatic progenitor cells.

For example, the pancreas progenitors may typically first occur as discrete Pdx1-positive cell clusters representing about 20 to 50% of the total cells in culture,

In an embodiment, the duration of said step (b) of the present methods may be between about 4 days and about 12 days, preferably between about 6 days and about 10 days, more preferably about 7 or 8 days.

Pancreatic progenitor cells as intended herein preferably comprise the expression of any one of, more preferably any two or more of, even more preferably all of Pdx1 (pancreatic and duodenal homeobox 1), Foxa2, Sox9 (SRY(sex determining region Y)-box 9) and Hlxb9 (homeo box HB9). In an embodiment, the pancreatic progenitor cells as intended herein may comprise at least the expression of Pdx1, preferably in conjunction with the expression of any one or more or all of Foxa2, Sox9 and Hlxb9. Also preferably, the pancreatic progenitor cells as intended herein may be negative for the expression of albumin (ALB) or α-fetoprotein (AFP), more preferably may be negative for the expression of both ALB and AFP.

A cell population comprising pancreatic progenitor cells as intended herein may comprise, without limitation, at least about 5%, at least about 10%, preferably at least about 15%, more preferably at least about 20% and even more preferably at least about 30% of said pancreas progenitor cells, *e.g*., of cells comprising the expression of any one of, more preferably any two or more of, even more preferably all of Pdx1, Foxa2, Sox9 and Hlxb9; or of cells comprising at least the expression of Pdx1, preferably in conjunction with the expression of any one or more or all of Foxa2, Sox9 and Hlxb9. Greater proportions of pancreatic progenitor cells in such cell populations are also envisaged, e.g., at least about 50%, at least about 60%, at least about 70%, at least about 80%, at least about 90%, at least about 95% or even a cell population consisting of or consisting substantially of the pancreatic progenitor cells as intended herein.

The invention also teaches the maintenance, proliferation and further differentiation of the pancreatic progenitor cells as taught above or as obtainable or directly obtained by the herein taught methods. An embodiment may expose the pancreatic progenitors to one or more agents that induce proliferation of said pancreatic progenitors, more preferably of Pdx1+ pancreatic progenitors. A further embodiment may expose the pancreatic progenitors to one or more agents that induce endocrine differentiation of said pancreatic progenitors, more preferably of Pdx1+ pancreatic progenitors.

In particular, a further aspect provides an *in vitro* method for producing pancreatic endocrine progenitor cells or a cell population comprising such, comprising the step of (herein denoted as step (c)) exposing the pancreatic progenitor cells or cell population comprising such cells as taught herein, or as obtainable or directly obtained by the methods taught above, to any one or more or all of an agonist of FGF signalling, an antagonist of Notch pathway and an agonist of glucagon-like peptide 1 (Glp-1) receptor.

In embodiments, the agonist of FGF signalling may be a growth factor selected from the FGF family. Preferably, the agonist of FGF signalling may be FGF-10.

In embodiments, the antagonist of Notch pathway is chosen from the group comprising or consisting of gamma secretase/presenilin inhibitors, recombinant notch extracellular domain, activators of notch intracellular domain degradation (SEL-10), and antibodies to notch receptor ligands including delta, serrate, jagged. Preferably the antagonist of Notch pathway may be a gamma secretase inhibitor, more preferably the gamma secretase inhibitor compound E.

In embodiments, the agonist of Glp-1 receptor may be exendin-4.

Preferably, the cells in said step (c) of the present methods are exposed at least to the agonist of FGF signalling and the antagonist of Notch pathway. More preferably, the cells in said step (c) of the present methods are exposed to all of the agonist of FGF signalling, the antagonist of Notch pathway and the agonist of Glp-1 receptor.

In an embodiment, the cells in said step (c) of the present methods may be exposed to any two or more of the agonist of FGF signalling, the antagonist of Notch pathway and the agonist of Glp-1 receptor simultaneously or sequentially in any order. Preferably, the cells in said step (c) of the present methods may be exposed to any two or more of the agonist of FGF signalling, the antagonist of Notch pathway and the agonist of Glp-1 receptor simultaneously, more preferably simultaneously and throughout the duration of said step (c).

The duration of step (c) may be generally chosen to be adequate to allow for the pancreatic endocrine progenitors to form. Said adequate duration may be determined by detecting the presence (*e.g*., by detecting the expression of corresponding markers) of pancreatic endocrine progenitor cells in the resulting cell population. By means of example, an adequate duration may be concluded when the resulting cell population comprises at least about 5%, at least about 10%, preferably at least about 15%, more preferably at least about 20% and even more preferably at least about 30%, even more preferably at least about 40%, at least about 50%, at least about 60%, yet more preferably at least about 70%, still more preferably at least about 80% or more of pancreatic endocrine progenitor cells.

For example, the pancreatic endocrine progenitors may typically occur as large Pdx1-positive cell monlayers or aggregates, representing about 40 to 90% of the total cells in culture, more typically at least 70%,

In an embodiment, the duration of said step (c) may be between about 1 day and about 8 days, preferably between about 2 days and about 6 days, more preferably between about 3 days and about 5 days, even more preferably about 4 days.

Pancreatic endocrine progenitor cells as intended herein preferably comprise the expression of any one of, more preferably any two or more of, even more preferably all of Pdx1, Foxa2, Sox9, Hlxb9, Hnf6 (hepatocyte nuclear factor-6), Nkx6.1 (NK6 homeobox 1) and Ngn3 (neurogenin 3). In an embodiment, pancreatic endocrine progenitor cells as intended herein may comprise the expression of Pdx1, Foxa2, Sox9 and of any one of, more preferably any two or more of, even more preferably all of Hlxb9, Hnf6, Nkx6.1 and Ngn3. In an embodiment, pancreatic endocrine progenitor cells as intended herein may comprise at least the expression of Ngn3, preferably in conjunction with one or more or all of Pdx1, Foxa2, Sox9, Hlxb9, Hnf6 and Nkx6.1. By means of example, the pancreatic endocrine progenitor cells may comprise the expression of at least Pdx1 and Ngn3. Also preferably, the pancreatic endocrine progenitor cells may be negative for the expression of ALB or AFP, more preferably may be negative for the expression of both ALB and AFP. Also typically, the pancreatic endocrine progenitors may be negative for or do not express substantial amounts of the pancreas enzyme and hormones amylase, insulin, and glucagon,

A cell population comprising pancreatic endocrine progenitor cells as intended herein may comprise, without limitation, at least about 10%, at least about 15%, at least about 20%, at least about 30%, at least about 40%, at least about 50%, at least about 60%, at least about 70%, at least about 80% of said pancreatic endocrine progenitor cells, *e.g*., of cells comprising the expression of any one of, more preferably any two or more of, even more preferably all of Pdx1, Foxa2, Sox9, Hlxb9, Hnf6, Nkx6.1 and Ngn3; or comprising at least the expression of Ngn3, preferably in conjunction with one or more or all of Pdx1, Foxa2, Sox9, Hlxb9, Hnf6 and Nkx6.1; or comprising at least the expression of Pdx1 and Ngn3. Even greater proportions of pancreatic endocrine progenitor cells in such cell populations are envisaged, *e.g*., at least about 90%, at least about 95% or even a cell population consisting of or consisting substantially of the pancreatic endocrine progenitor cells as intended herein.

In preferred embodiments, the pancreatic progenitor cells or more preferably the pancreatic endocrine progenitor cells as intended herein are capable of further differentiation into beta-cells *in vivo* (*e.g.,* following transplantation) or *in vitro.*

Hence, the present invention also discloses an *in vitro* method for producing pancreatic endocrine progenitor cells or a cell population comprising such, comprising the steps of: (a) providing cells having definitive endoderm germ layer phenotype; (b) exposing the cells of (a) to an antagonist of BMP pathway and to one or both of, preferably both of, an antagonist of hedgehog pathway and an agonist of retinoid signalling, thereby producing pancreatic progenitor cells or a cell population comprising such; and (c) exposing said pancreatic progenitor cells or cell population comprising such of (b) to any one, more or all of an agonist of FGF signalling, an antagonist of Notch pathway and an agonist of Glp-1 receptor. The preferred features described above for steps (a), (b) and (c) also apply to this aspect.

The herein taught methods allow to produce pancreatic progenitors and pancreatic endocrine progenitors and cell populations comprising such progenitor cell types, *e.g*., cell populations enriched or substantially homogeneous for said progenitor cell types. When needed, such cell populations may be collected or harvested and said pancreatic progenitors or pancreatic endocrine progenitors may be further enriched or isolated there from on the basis of their distinctive characteristics (such as, for example, their marker expression as defined above) using methods generally known in the art (*e.g*., FACS).

Accordingly, in further aspects the invention provides isolated pancreatic progenitors and pancreatic endocrine progenitors, as well as isolated cell cultures and cell populations comprising said progenitor cell types, as disclosed above or as obtainable or directly obtained by the herein taught methods.

The present progenitor cells and cell populations comprising such progenitors may be employed in various applications, such as *inter alia* in pharmacological, toxicological or genetic screening assays; in cellular models of pancreatic development and function (*e.g*., as cellular models of generation and function of beta-cells or other endocrine pancreatic cell types), in cellular models of pancreatic impairment, dysfunction or diseases; in cell-replacement therapies of pancreatic impairment, dysfunction or diseases; etc.

A further aspect thus provides an *in vitro* method for producing pancreatic endocrine cells, preferably beta-cells, comprising exposing the pancreatic progenitors or, preferably, the pancreatic endocrine progenitors as taught herein to one or more agents or conditions capable of affecting differentiation of said pancreatic progenitors or pancreatic endocrine progenitors into said pancreatic endocrine cells, preferably beta-cells. Also provided are isolated pancreatic endocrine cells, preferably beta-cells, obtainable or directly obtained by this method. Preferably, said beta-cells are functional, *i.e.,* insulin-producing, more preferably insulin-producing and glucose responsive.

Hence, the invention also provides isolated pancreatic progenitor cells or a cell population comprising such, or isolated pancreatic endocrine progenitor cells or a cell population comprising such, or isolated pancreatic endocrine cells or a cell population comprising such, preferably beta-cells or a cell population comprising such, obtainable or directly obtained by the above taught methods.

In related aspects, the invention provides compositions, including pharmaceutical formulations, comprising: (i) isolated pancreatic progenitor cells or a cell population comprising such progenitor cells as taught herein; and/or (ii) isolated pancreatic endocrine progenitor cells or a cell population comprising such progenitor cells as taught herein; and/or (iii) isolated pancreatic endocrine cells, preferably beta-cells, obtainable or directly obtained by further differentiating the progenitors or cell populations of (i) or (ii) as taught herein.

Hence, in an aspect the invention also provides a pharmaceutical composition comprising:
- isolated pancreatic progenitor cells or a cell population comprising such, and/or isolated pancreatic endocrine progenitor cells or a cell population comprising such, and/or isolated pancreatic endocrine cells or a cell population comprising such, preferably beta-cells or a cell population comprising such, obtainable or directly obtained by the above taught methods, and
- one or more pharmaceutically acceptable carriers.

Further aspects relate to prophylactic and therapeutic uses (*e.g*., in conditions or diseases associated with impairment or dysfunction of endocrine pancreas, or in conditions or diseases associated with impaired beta-cell function, or in conditions or diseases associated with impaired glucose homeostasis, such as, for example, in prediabetes or diabetes, *etc*.), or non-medical uses, of any of the herein taught isolated cells (such as pancreatic progenitors, pancreatic endocrine progenitors or pancreatic endocrine cells such as beta-cells), isolated cell populations comprising such cells, and compositions including pharmaceutical compositions comprising said isolated cells or cell populations.

Hence, the invention also contemplates isolated pancreatic progenitor cells or a cell population comprising such, and/or isolated pancreatic endocrine progenitor cells or a cell population comprising such, and/or or isolated pancreatic endocrine cells or a cell population comprising such, preferably beta-cells or a cell population comprising such, obtainable or directly obtained by the above taught methods, for use in therapy or for use as a medicament, for example for use in the treatment of conditions or diseases associated with impairment or dysfunction of endocrine pancreas, for example for use in the treatment of conditions or diseases associated with impaired beta-cell function, for example for use in the treatment of conditions or diseases associated with impaired glucose homeostasis, for example in the treatment of prediabetes or diabetes.

These and further aspects and preferred embodiments of the invention are described in the following sections and in the appended claims.

### DESCRIPTION OF FIGURES

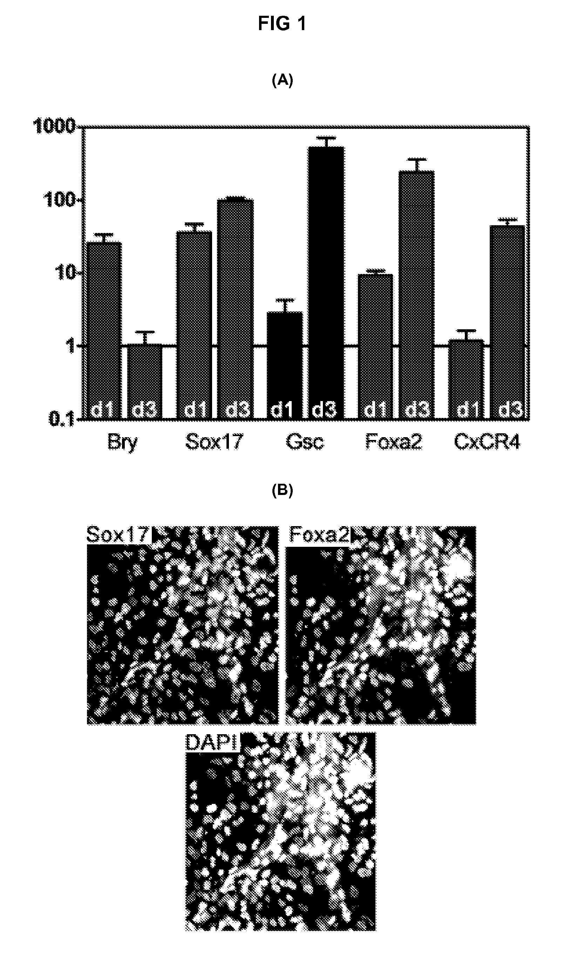

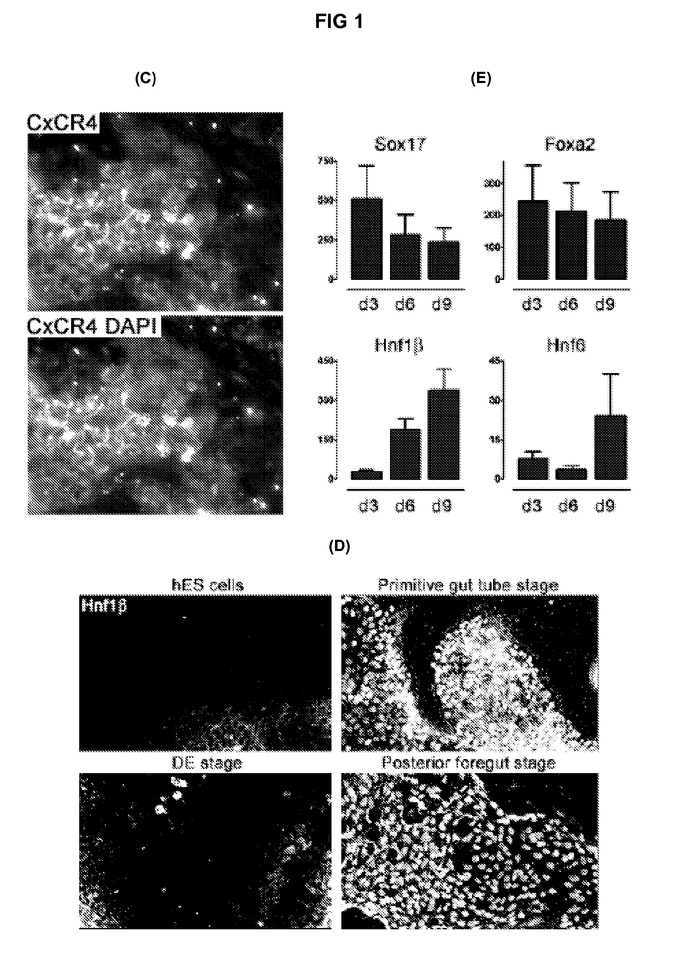

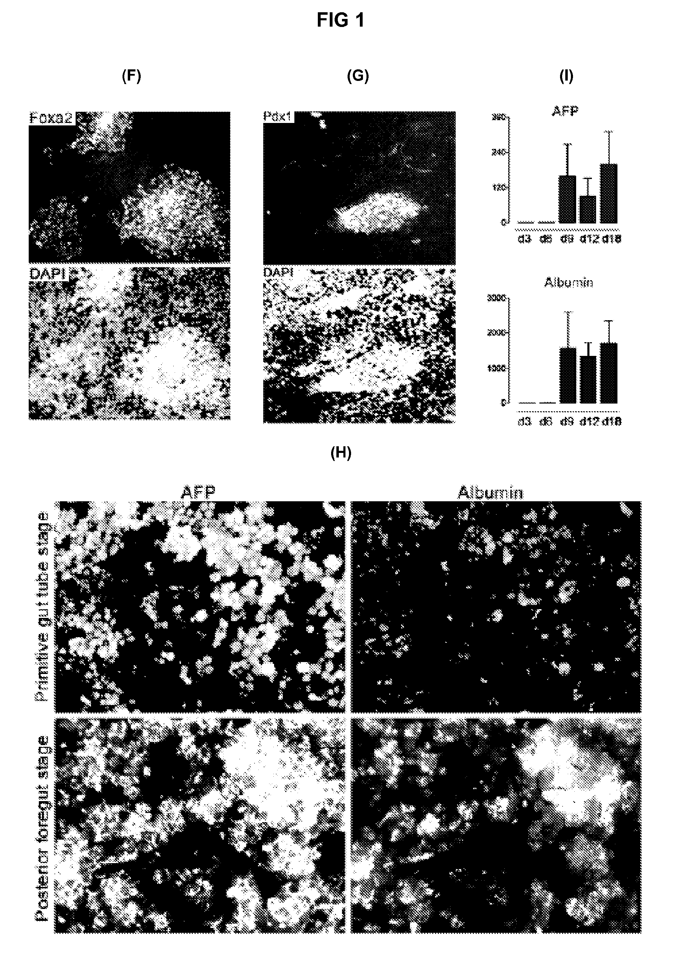

**Figure 1****: Definitive endoderm derived from human embryonic stem cells give rise to hepatoblast-like cells when exposed to FGF ligands.** Human embryonic stem cells were cultured on mouse embryonic fibroblasts and treated with Activin A (100 ng/ml) and Wnt3a (25 ng/ml) for one day, then with Activin A and foetal bovine serum (0.2%) for 2 days. Differentiated cells were examined on day 3 for the expression of definitive endoderm markers by PCR and immunocytochemistry. The mesoderm marker Bry was transiently expressed after one day of induction, and decreased concomitantly with increasing expression of endoderm markers Gsc, Sox17, Foxa2 and CxCR4 (**A**). Expression levels were normalized versus undifferentiated hES cells set at 1. The colocalization of Foxa2 with Sox17, which marks the definitive endoderm, was confirmed by immunoreactivity with corresponding antibodies (**B**). Similarly, membrane expression of CxCR4 was detected at the same stage (**C**). Exposure of these definitive endoderm cells to FGF10 (50 ng/ml) and KAAD-cyclopamine (250 nM) for at least 3 days resulted in a major population of cells expressing Hnf1b, which marks the primitive gut tube (**D**)**.** The definitive endoderm marker Sox17 started to decrease, while Hnf1b further increased in the next stage and Hnf6 was induced (**E**)**.** At the posterior foregut stage, cluster of Foxa2-positive (**F**) and Pdx1-positive (**G**) cells could be identified, but at much lower frequency for Pdx1. In contrast to Pdx1, many cells expressed alpha-fetoprotein (AFP) and low levels of albumin (ALB), representing early hepatic progenitors or hepatoblasts (**H**). The sharp increase in alpha-fetoprotein and albumin gene expression between the PGT and PFG stages is highlighted by PCR analysis (**I**)**.**

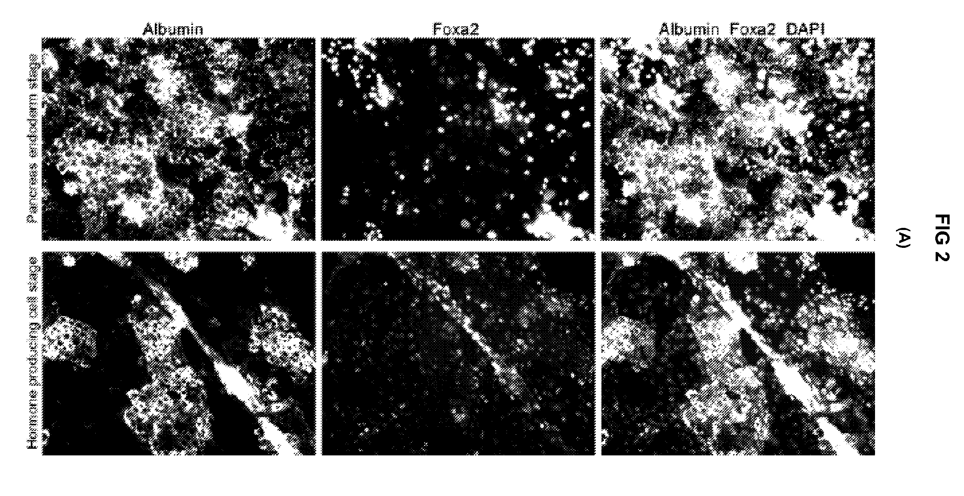

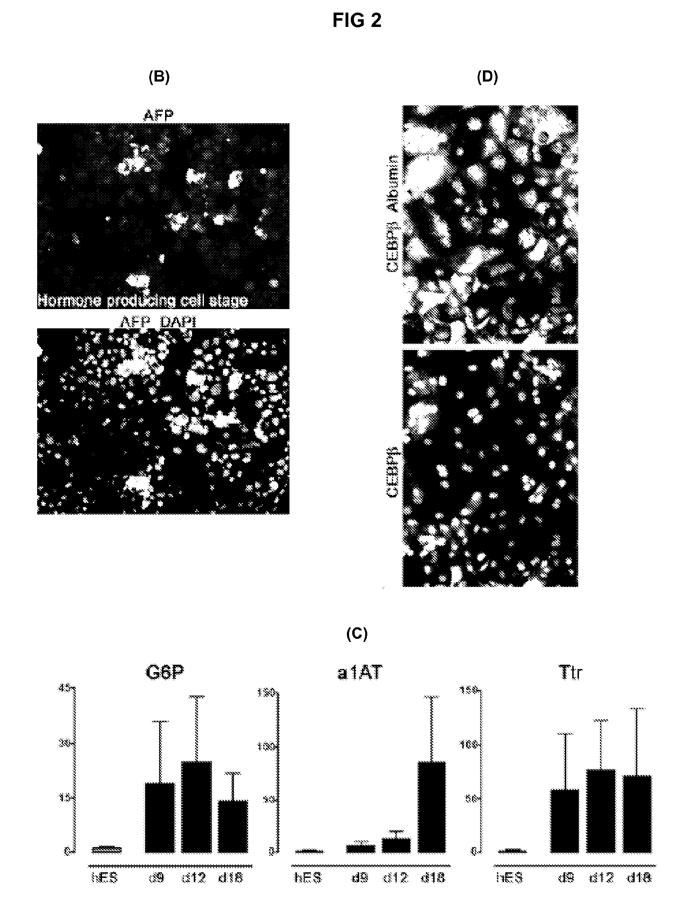

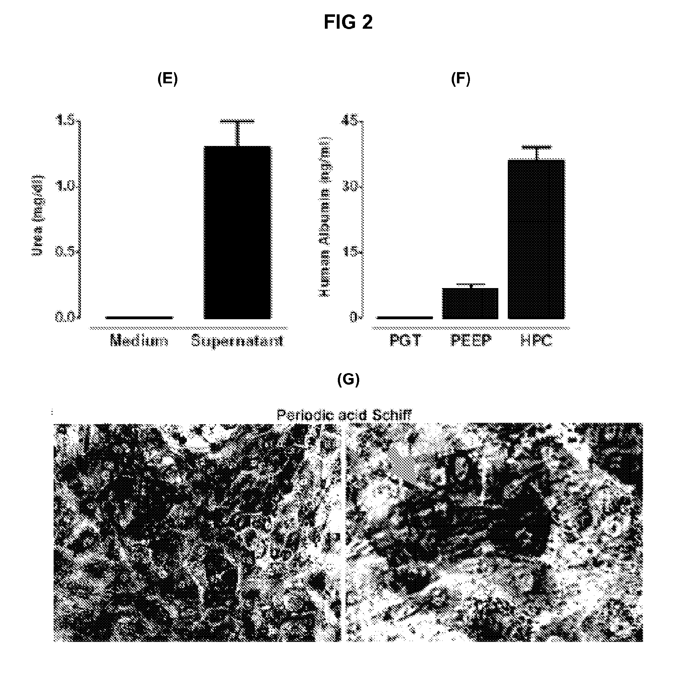

**Figure 2****: Hepatoblast-like cells further differentiate into functional equivalents of hepatocytes.** Further culture of hepatoblast-like cells for at least 3 days led to a reduced level of AFP and an increased expression of ALB, which was co-localized with Foxa2 (**A**)**.** At the end of culture by day 21, AFP expression was limited to a minority of cells (**B**)**.** Quantitative PCR analysis indicated upregulation of other hepatic markers such as glucose-6-phosphatase (G6P), alpha-1-antitrypsine (a1AT) and transthyretin (Ttr)(**C**). Additionally, differentiated ALB-positive cells also co-expressed CEBPβ in their nuclei (**D**)**.** The sequential expression of AFP, ALB, Foxa2, alpha-1-antitrypsine and CEBPβ suggests a progressive transition from hepatoblasts to hepatocytes in culture. To examine the functional status of these hepatocytes-like cells at the end of culture, they were treated overnight with 10mM ammonium chloride and urea was measured in the culture medium. Data indicate that the obtained hepatocytes synthesized and secreted urea (**E**). Media were collected at intervals during culture and analyzed by ELISA for human albumin. Data also indicate a progressive increase in the amount of albumin secreted in the medium (**F**). Finally, another function of mature hepatocytes is to store glycogen. Using periodic acid Schiff staining, we provide evidence that human embryonic stem cells-derived hepatocytes, some of which were binucleated (arrow) also stored glycogen (**G**).

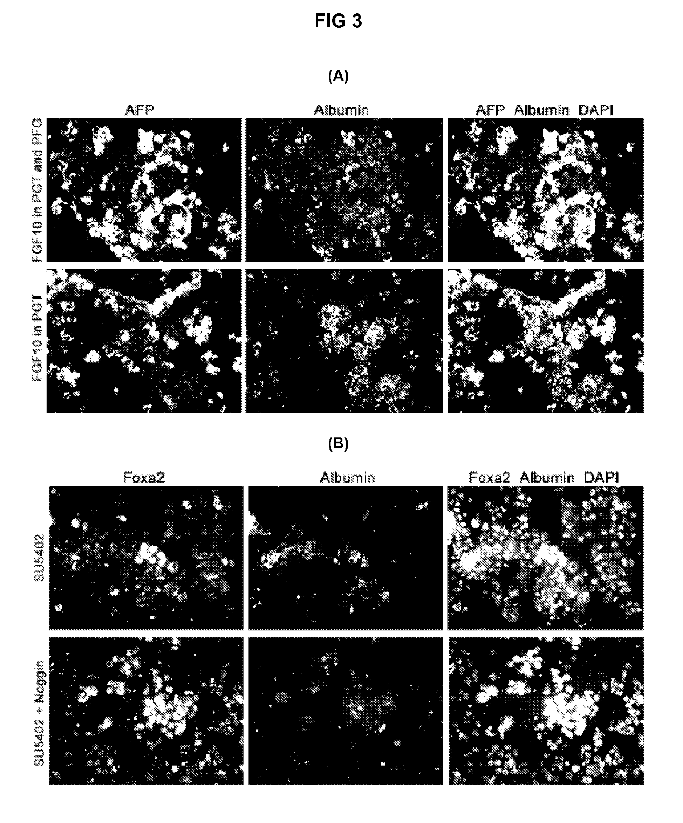

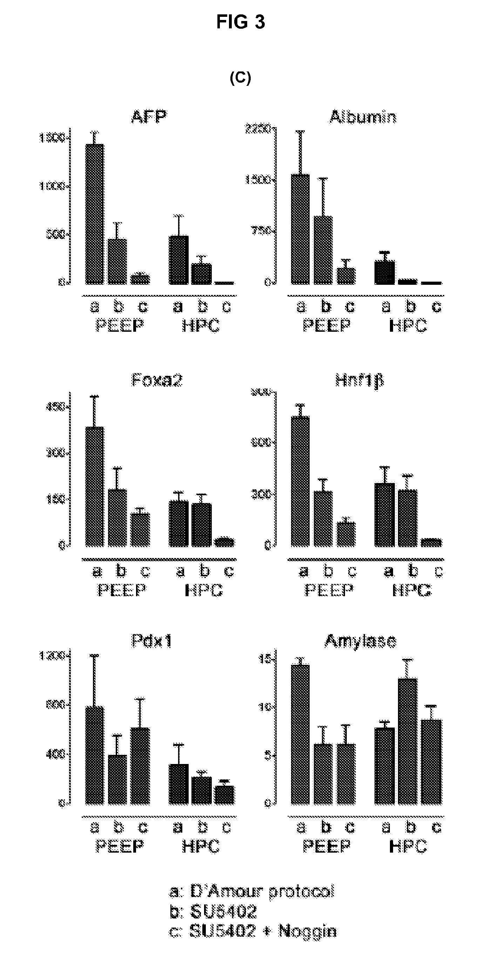

**Figure 3****: Antagonism with BMP and FGF pathways abolishes hepatocytes differentiation**. Early removal of FGF10 from cultures accelerated the loss of AFP in differentiating hepatoblasts, and resulted mainly in two populations: AFP+ALB- and AFP-ALB+. This confirms the role of FGF10 in the maintenance of hepatoblasts (**A**). Exposure of definitive endoderm cells to SU5402 (250 nM), an inhibitor of FGF-receptor attenuates albumin expression by the pancreas endoderm stage, whereas combined treatment with SU5402 and the BMP antagonist noggin (100 ng/ml) almost completely abolished albumin expression **(B).** The effects of SU5402 and noggin are confirmed at the transcript level for other genes. Remarkably, inhibition of hepatogenesis was not associated with any improvement of Pdx1 expression, possibly but without limitation because upstream factors such as Hnf1β and Foxa2 were also negatively affected (**C**).

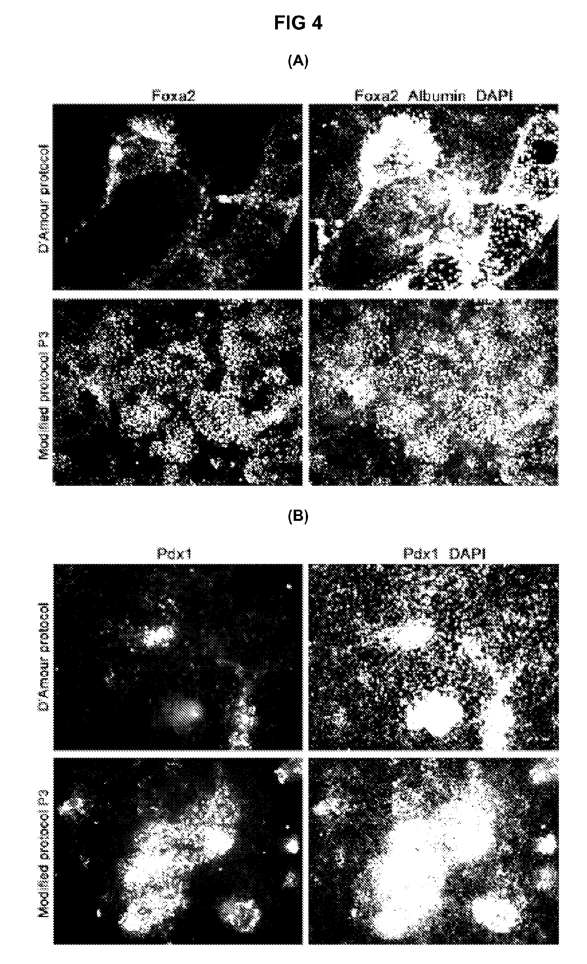

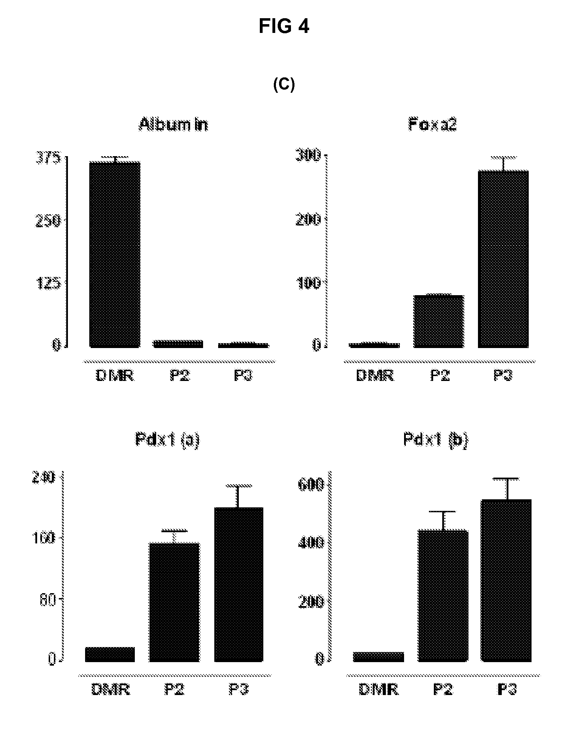

**Figure 4****: Generation of early pancreas progenitors expressing Foxa2 and Pdx1.** Combined application of BMP antagonist, hedgehog antagonist and retinoid acid without supplemented FGF ligands and without suppressing the endogenous FGF signalling allows for the establishment of cells expressing much higher levels of Foxa2 and very low, if any albumin (**A**). Similarly, this treatment allowed for the differentiation of much frequent and larger clusters of Pdx1-expressing cells (**B**). Quantitative PCR analyses confirmed these observations, showing down-regulation of albumin and up-regulation of Foxa2 and Pdx1 (evaluated with 2 different primer sets) in both protocols P2 and P3 (**C**).

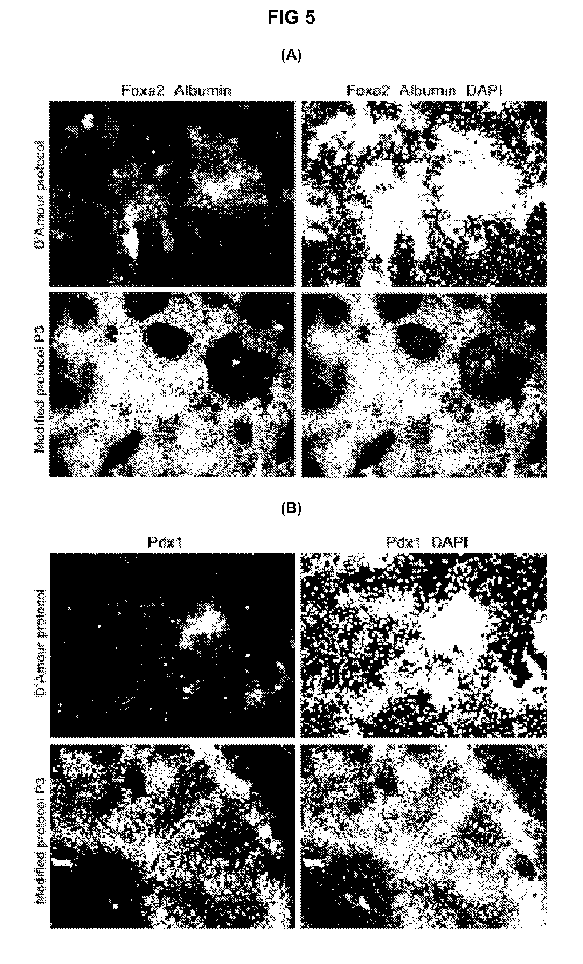

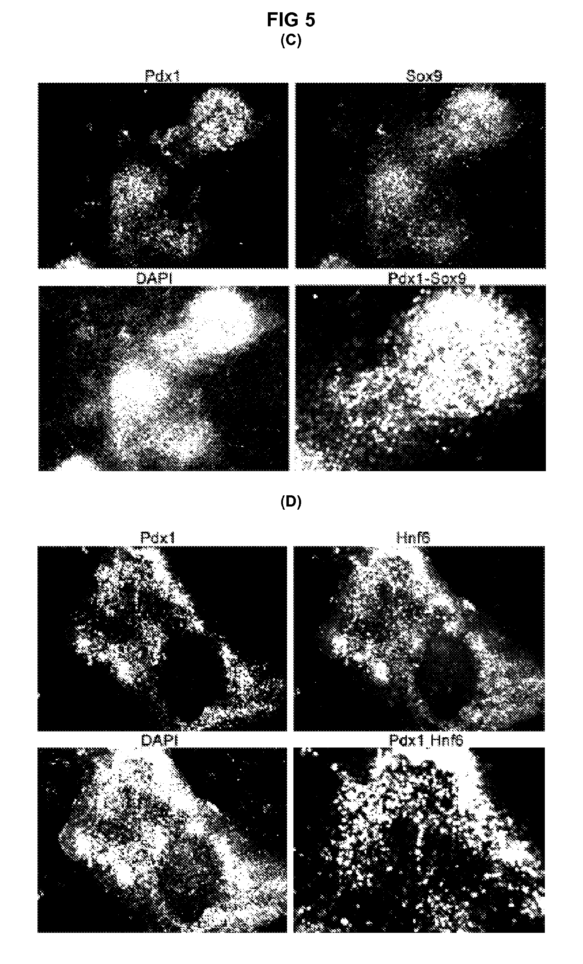

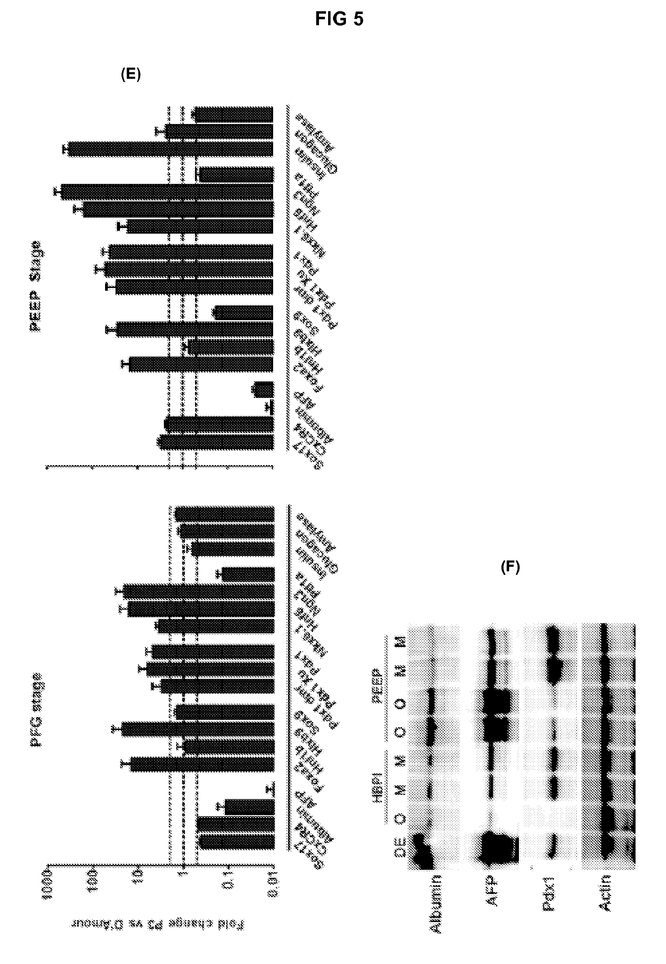

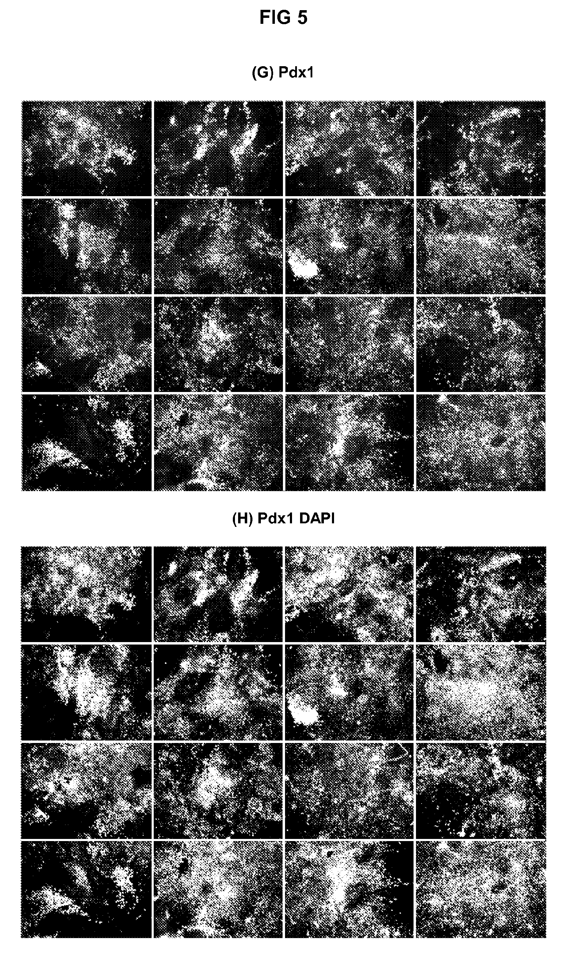

**Figure 5****: Generation of late pancreas progenitors expressing Pdx1 as well as higher levels of the endocrine progenitor markers Hnf6 and Ngn3.** Finally, exposure of such early pancreas progenitors to FGF signalling and Notch inhibitor induced a further increase in Foxa2 expression and secured the loss of albumin observed in the HBPI stage (**A**). Concomitantly, large proportions of Pdx1-positive cells were frequently observed in these cultures, representing up to 80% of cells (**B**). The Pdx1-positive cells observed in PEEP stage of protocol P3 also expressed higher levels of other transcription factors characteristic of pancreatic and endocrine progenitor cells including Sox9 (**C**) and Hnf6 **(D).** Quantitative PCR analysis of gene expression in protocol P3 versus D'Amour protocol at two time points also indicated higher levels of many pancreas and endocrine transcription factors, in contrast to lower levels of albumin and AFP transcripts (**E**). The demonstration of the beneficial effects of the new method is evidenced in the protein analysis by western blot, showing a shift from AFP-ALB expression to Pdx1 expression when the mentioned combination of growth factors and chemicals is used (**F**). Composite picture made of several fields from a typical culture well of the preferred embodiment in which at least 80% of cells are Pdx1-expressing cells (**G, H**).

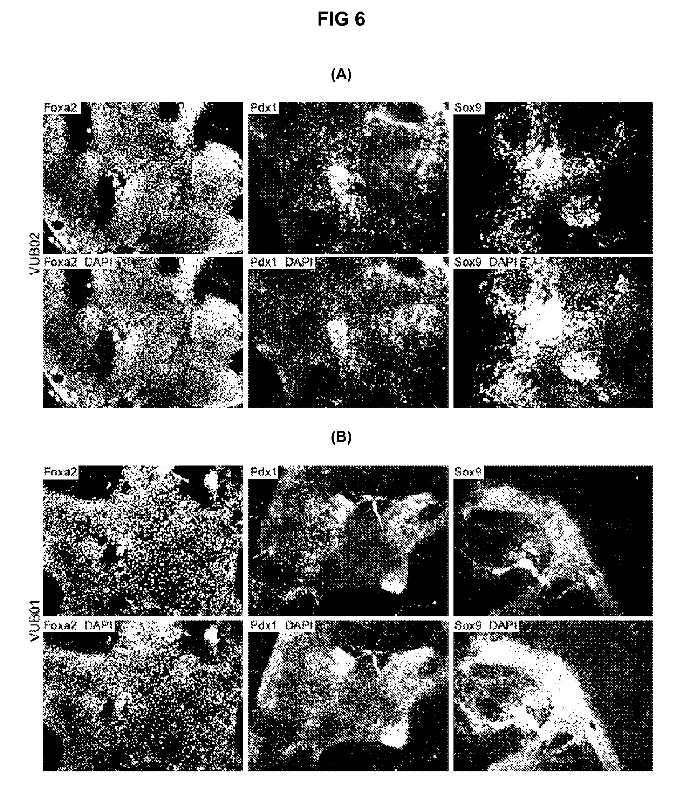

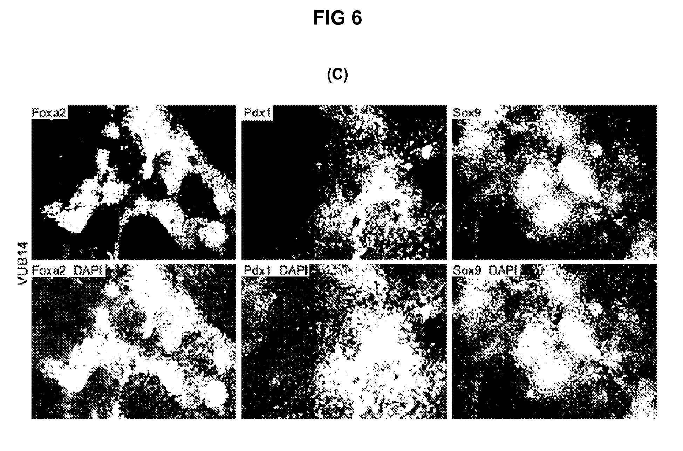

**Figure 6****: Data indicating reproduction of the findings in three additional human embryonic stem cell lines.** Immunoreactivity analysis showing expression of Foxa2, Pdx1 and Sox9 at the pancreas and endocrine progenitor stage upon treatment of the cells according to the methods described in the present invention. Data are shown for VUB02 (**A**), VUB01 (**B**) and VUB14 (**C**) human embryonic stem cell lines.

### DETAILED DESCRIPTION OF THE INVENTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

All documents cited in the present specification are hereby incorporated by reference in their entirety.

Unless otherwise specified, all terms used in disclosing the invention, including technical and scientific terms, have the meaning as commonly understood by one of ordinary skill in the art to which this invention belongs. By means of further guidance, term explanations or definitions may be included to better appreciate the teaching of the present invention. When certain terms are explained or defined in connection with a particular aspect or embodiment, such connotation is meant to apply throughout this specification, *i.e.,* also for other aspects or embodiments, unless otherwise specified or unless the context clearly dictates otherwise.

For general methods relating to the invention, reference is made *inter alia* to well-known textbooks, including, *e.g*., "Molecular Cloning: A Laboratory Manual, 2nd Ed." (Sambrook et al., 1989), Animal Cell Culture (R. I. Freshney, ed., 1987), the series Methods in Enzymology (Academic Press), Gene Transfer Vectors for Mammalian Cells (J. M. Miller & M. P. Calos, eds., 1987); "Current Protocols in Molecular Biology and Short Protocols in Molecular Biology, 3rd Ed." (F. M. Ausubel et al., eds., 1987 & 1995); Recombinant DNA Methodology II (R. Wu ed., Academic Press 1995).

General techniques in cell culture and media uses are outlined *inter alia* in Large Scale Mammalian Cell Culture (Hu et al. 1997. Curr Opin Biotechnol 8: 148); Serum-free Media (K. Kitano. 1991. Biotechnology 17: 73); or Large Scale Mammalian Cell Culture (Curr Opin Biotechnol 2: 375, 1991).

For further elaboration of general techniques useful in the practice of this invention, the practitioner can refer to standard textbooks and reviews in cell biology, tissue culture, and embryology. Included are *inter alia* "Teratocarcinomas and embryonic stem cells: A practical approach" (E. J. Robertson, ed., IRL Press Ltd. 1987); "Guide to Techniques in Mouse Development" (P. M. Wasserman et al. eds., Academic Press 1993); "Embryonic Stem Cells: Methods and Protocols" (Kursad Turksen, ed., Humana Press, Totowa N.J., 2001); "Embryonic Stem Cell Differentiation in Vitro" (M. V. Wiles, Meth. Enzymol. 225: 900, 1993); "Properties and uses of Embryonic Stem Cells: Prospects for Application to Human Biology and Gene Therapy" (P. D. Rathjen et al., al., 1993). Differentiation of stem cells is reviewed, *e.g*., in Robertson. 1997. Meth Cell Biol 75: 173; Roach and McNeish. 2002. Methods Mol Biol 185: 1-16; and Pedersen. 1998. Reprod Fertil Dev 10: 31.

Disclosed herein are *in vitro* methods and protocols for producing desired cells and cell populations. The term "*in vitro*" generally denotes outside, or external to, a body, e.g., an animal or human body. The term "*ex vivo*" typically refers to tissues or cells removed from a body, e.g., an animal or human body, and maintained or propagated outside the body, e.g., in a culture vessel. The term "*in vitro*" as used herein should be understood to include *"ex vivo*".

The methods may typically involve culturing (*e.g*., maintaining, propagating and/or differentiating) the cells and cell populations taught herein in the presence of cell or tissue culture media as known *per se,* such as for example using liquid or semi-solid (*e.g*., gelatinous), and preferably liquid cell or tissue culture media. Such culture media can desirably sustain the maintenance (*e.g*., survival, genotypic, phenotypic and/or functional stability) and propagation of the cells or cell populations.

Such culture media can may where desired be permissive to differentiation of the cultured cells or cell populations. A medium which is permissive to a differentiation of given cells or cell populations may for example not contain components, in sufficient quantity, which would suppress said differentiation of the cells or would cause maintenance and/or proliferation of the cells in undifferentiated or substantially undifferentiated state. By means of illustration, when differentiation of pluripotent stem cells such as mPS or hPS is desired, components absent from the medium may include leukaemia inhibitory factor (LIF), basic fibroblast growth factor (b-FGF), and/or embryonic fibroblast feeders or conditioned medium of such feeders, depending on the particular stem cell type. In another example, when differentiation of definitive endoderm cells to pancreatic progenitors as intended herein is desired, components absent from the medium may include an exogenous (exogenously added) agonist of FGF signalling.

Typically, a medium may comprise a basal medium formulation as known in the art. Various basal media formulations are available, *e.g*., from the American Type Culture Collection (ATCC) or from Invitrogen (Carlsbad, California). By means of example and not limitation, basal media formulations may include Eagle's Minimum Essential Medium (MEM), Dulbecco's Modified Eagle's Medium (DMEM), alpha modified Minimum Essential Medium (alpha-MEM), Basal Medium Essential (BME), F-12 Nutrient Mixture (Ham), Iscove's Modified Dulbecco's Medium (IMDM), RPMI 1640 Medium, and modifications and/or combinations thereof. Compositions of basal media are generally known in the art and it is within the skill of one in the art to modify or modulate concentrations of media and/or media supplements as necessary for the cells cultured.

Such basal media formulations contain ingredients necessary for mammalian cell development, which are known *per se.* By means of illustration and not limitation, these ingredients may include inorganic salts (in particular salts containing Na, K, Mg, Ca, CI, P and possibly Cu, Fe, Se and Zn), physiological buffers (*e.g*., HEPES, bicarbonate or phosphate buffers), pH indicators, sources of carbon (*e.g*., glucose, sodium pyruvate, sodium acetate), and may further also comprise reducing agents or antioxidants (*e.g*., glutathione), vitamins, nucleotides, nucleosides and/or nucleic acid bases, ribose, deoxyribose, amino acids, etc.

For use in culture, basal media can be supplied with one or more further components. For example, additional supplements can be used to supply the cells with further necessary trace elements and substances for optimal growth and expansion. Further antioxidant supplements may be added, *e.g*., β-mercaptoethanol. While many basal media already contain amino acids, some amino acids may be supplemented later, e.g., L-glutamine, which is known to be less stable when in solution. A medium may be further supplied with antibiotic and/or antimycotic compounds, such as, typically, mixtures of penicillin and streptomycin, and/or other compounds, exemplified but not limited to, amphotericin, ampicillin, gentamicin, bleomycin, hygromycin, kanamycin, mitomycin, mycophenolic acid, nalidixic acid, neomycin, nystatin, paromomycin, polymyxin, puromycin, rifampicin, spectinomycin, tetracycline, tylosin, and zeocin.

Lipids and lipid carriers can also be used to supplement cell culture media. Such lipids and carriers can include, but are not limited to cyclodextrin, cholesterol, linoleic acid conjugated to albumin, linoleic acid and oleic acid conjugated to albumin, unconjugated linoleic acid, linoleic-oleic-arachidonic acid conjugated to albumin, oleic acid unconjugated and conjugated to albumin, among others. Albumin can similarly be used in fatty-acid free formulations.

Where desirable, media may be further supplemented with plasma or serum, more typically with serum.

The term "plasma" is as conventionally defined. Plasma is usually obtained from an isolated sample of whole blood, provided or contacted with an anticoagulant, (*e.g*., heparin, citrate, oxalate or EDTA). Subsequently, cellular components of the blood sample are separated from the liquid component (plasma) by an appropriate technique, typically by centrifugation.

The term "serum" is as conventionally defined. Serum can be usually obtained from an isolated sample of whole blood by first allowing clotting to take place in the sample and subsequently separating the so formed clot and cellular components of the blood sample from the liquid component (serum) by an appropriate technique, typically by centrifugation. Clotting can be facilitated by an inert catalyst, *e.g*., glass beads or powder. Alternatively, serum can be obtained from plasma by removing the anticoagulant and fibrin.

The isolated plasma or serum can be used directly , or they can be appropriately stored for later use (*e.g*., for shorter time periods, e.g., up to about 1-2 weeks, at a temperature above the respective freezing points of plasma or serum, but below ambient temperature, this temperature will usually be about 4°C to 5°C; or for longer times by freeze storage, usually at between about -70°C and about -80°C).

The isolated plasma or serum can be heat inactivated as known in the art, particularly to remove the complement. Optionally, the plasma or serum may also be sterilised prior to storage or use, using conventional microbiological filters, preferably with pore size of 0.2µm or smaller.

Suitable sera or plasmas may include human serum or plasma, or serum or plasma from non-human animals, preferably non-human mammals, such as, *e.g*., non-human primates (*e.g*., lemurs, monkeys, apes), foetal or adult bovine, horse, porcine, lamb, goat, dog, rabbit, mouse or rat serum or plasma, *etc*., or combinations thereof.

Bovine serum or plasma, preferably foetal bovine (calf) serum or plasma, more preferably foetal bovine (calf) serum (FCS or FBS) may be commonly used.

Serum or plasma may , be commonly comprised in a medium at a proportion (volume of plasma or serum / volume of medium) between about 0.1% and about 30%, preferably between about 0.5% and about 15%, *e.g*., about 5 or about 10 volume %.

Alternatively, media may be serum-free. Serum-free media may be typically supplemented with defined serum-replacements, which are known *per se* (*e.g.,* WO 98/30679) and are commercially available.

Cells and cell populations as intended herein preferably encompass animal cells and cell populations, more preferably refer to mammalian cells and cell populations. The terms "mammal" and "mammalian" refer to any animal classified as such, including, but not limited to, humans, domestic and farm animals, zoo animals, sport animals, pet animals, companion animals and experimental animals, such as, for example, mice, rats, hamsters, rabbits, dogs, cats, guinea pigs, cattle, cows, sheep, horses, pigs and primates, *e.g*., monkeys and apes. In embodiments, the cells and cell populations as intended herein may be human, or may be non-human, such as, *e.g*., non-human animal or preferably non-human mammalian cells and cell populations.

The present specification further concerns certain isolated cells and cell populations. The term "isolated" with reference to a particular component generally denotes that such component exists in separation from - for example, has been separated from or prepared and/or maintained in separation from - one or more other components of its natural environment. The term "isolated" in connection to cells and cell populations may particularly denote that such cells or cell populations do not form part of a body, *e.g*., an animal or human body. For instance, isolated cells or cell populations may exist in separation from a body where they occur naturally.

The present specification concerns *inter alia* pancreatic progenitor cells, pancreatic endocrine progenitor cells, pancreatic endocrine cells, as well as cell populations comprising one or more of these cell types.

The terms "progenitor" or "progenitor cell" are synonymous and generally refer to an unspecialised or relatively less specialised and proliferation-competent cell which can under appropriate conditions give rise to at least one relatively more specialised cell type, such as *inter alia* to relatively more specialised progenitor cells or eventually to terminally differentiated cells, *i.e.,* fully specialised cells that may be post-mitotic. A progenitor cell may "give rise" to another, relatively more specialised cell when, for example, the progenitor cell differentiates to become said other cell without previously undergoing cell division, or if said other cell is produced after one or more rounds of cell division and/or differentiation of the progenitor cell.

Pancreatic progenitors, pancreatic endocrine progenitors and pancreatic endocrine cells as used herein denote increasingly advanced stages of pancreatic lineage differentiation, more particularly pancreatic endocrine lineage differentiation.

The terms "pancreatic progenitor" and "pancreatic progenitor cell" generally refer to a progenitor cell that can under appropriate conditions give rise to one or more pancreatic cell types, *i.e.,* to one or more cell types having - at least in part - the phenotype of native pancreatic cells. For example, a pancreatic progenitor cell may give rise to any one or more or all of exocrine, duct and endocrine pancreatic cell types. Preferably, a pancreatic progenitor cell may give rise to at least endocrine pancreatic cell types; more preferably to any one or more or all pancreatic endocrine cell types chosen from beta-cells, alpha-cells, delta-cells, PP-producing cells and epsilon cells; even more preferably at least to or only to beta-cells.

In an embodiment, a pancreatic progenitor cell may be committed to pancreatic differentiation, *i.e.,* may under appropriate conditions give rise predominantly or substantially exclusively to said one or more pancreatic cell types. For example, at least about 50%, at least about 60%, at least about 70%, at least about 80%, or at least about 90%, or even at least about 95% of so-differentiated cells may be of said one or more pancreatic cell types. In another embodiment, a pancreatic progenitor cell may be substantially not capable of differentiating along the hepatic lineage. For example, less than about 20%, preferably less than about 10%, more preferably less than about 5%, even more preferably less than about 1% or less of so-differentiated cells would display hepatic phenotype, such as hepatoblasts or hepatocyte phenotype (*e.g*., AFP and/or ALB expression).

The terms "pancreatic endocrine progenitor" and "pancreatic endocrine progenitor cell" generally refer to a progenitor cell that can under appropriate conditions give rise to one or more pancreatic endocrine cell types, *i.e.,* to one or more cell types having - at least in part - the phenotype of native pancreatic endocrine cells. For example, a pancreatic endocrine progenitor cell may give rise to any one or more or all pancreatic endocrine cell types chosen from beta-cells, alpha-cells, delta-cells, PP-producing cells and epsilon cells; more preferably at least to or only to beta-cells.

In an embodiment, a pancreatic endocrine progenitor cell may be committed to pancreatic endocrine differentiation, *i.e.,* may under appropriate conditions give rise predominantly or substantially exclusively to said one or more pancreatic endocrine cell types. For example, at least about 50%, at least about 60%, at least about 70%, at least about 80%, or at least about 90%, or even at least about 95% of so-differentiated cells may be of said one or more pancreatic endocrine cell types. In another embodiment, a pancreatic endocrine progenitor cell may be substantially not capable of differentiating along the hepatic lineage. For example, less than about 20%, preferably less than about 10%, more preferably less than about 5%, even more preferably less than about 1% or less of so-differentiated cells would display hepatic phenotype, such as hepatoblasts or hepatocyte phenotype (*e.g*., AFP and/or ALB expression). In another embodiment, a pancreatic endocrine progenitor cell may be substantially not capable of differentiating along a non-endocrine pancreatic lineage (e.g., an exocrine or duct lineage). For example, less than about 20%, preferably less than about 10%, more preferably less than about 5%, even more preferably less than about 1% or less of so-differentiated cells would display non-endocrine pancreatic lineage phenotype.

The term "pancreatic endocrine cell" as used herein refers to a cell having - at least in part - the phenotype of a native, specialised (*i.e.*, mature) pancreatic endocrine cell, such as, for example, a beta-cell, alpha-cell, delta-cell, PP-producing cell or epsilon cell, more preferably a beta-cell.

Preferably, said phenotype may encompass at least the ability of the cell to express a pancreatic hormone (*e.g*., insulin, glucagon, somatostatin, PP or ghrelin) normally expressed by a corresponding native, mature pancreatic endocrine cell. Also preferably, the expression of said hormone by the cell may be subject to regulation which is at least qualitatively and preferably also quantitatively equivalent to the regulation observed in the corresponding native, mature pancreatic endocrine cells.

By means of example, the term "beta-cell" or "beta-like cell" as used herein refers to a cell having - at least in part - the phenotype of a native, mature pancreatic beta-cell. Preferably, said phenotype may encompass at least the ability of the cell to express and preferably secrete insulin. Also preferably, the expression and secretion of insulin may be responsive to the presence and/or level (e.g., concentration) of glucose in the cell or contacting the cell.

Cells such as *inter alia* pancreatic progenitors, pancreatic endocrine progenitors or pancreatic endocrine cells may also be suitably characterised by their expressing or not expressing certain markers, such as, for example, nucleic acid and/or protein markers (*e.g*., enzymes, hormones, structural proteins, surface markers, *etc*.).

For example, pancreatic progenitor cells as intended herein may comprise the expression of any one or more or all of Pdx1, Foxa2, Sox9 and Hlxb9; preferably may comprise at least the expression of Pdx1; more preferably may comprise at least the expression of Pdx1, in conjunction with the expression of any one or more or all of Foxa2, Sox9 and Hlxb9; also preferably may comprise at least the expression of Pdx1 and Foxa2; also preferably may comprise at least the expression of Pdx1 and Foxa2, in conjunction with the expression of any one or both of Sox9 and Hlxb9. By means of example and without limitation, pancreatic progenitor cells as intended herein may thus comprise the expression of Pdx1, Foxa2 and Sox9; or may comprise the expression of Pdx1, Foxa2 and Hlxb9; or may comprise the expression of Pdx1, Foxa2, Sox9 and Hlxb9. Preferably, said pancreatic progenitor cells may be negative for the expression of ALB and/or AFP, more preferably for the expression of ALB and AFP.

For example, pancreatic endocrine progenitor cells as intended herein may comprise the expression of any one or more or all of Pdx1, Foxa2, Sox9, Hlxb9, Hnf6, Nkx6.1 and Ngn3; preferably may comprise at least the expression of Pdx1, Foxa2, Sox9 and of any one or more or all of Hlxb9, Hnf6, Nkx6.1 and Ngn3, for example may comprise the expression of Pdx1, Foxa2, Sox9 and Ngn3; also preferably may comprise at least the expression of Pdx1, Foxa2 and of any one or more or all of Hlxb9, Hnf6, Nkx6.1 and Ngn3, for example may comprise the expression of Pdx1, Foxa2 and Ngn3; also preferably may comprise at least the expression of Pdx1 and of any one or more or all of Hlxb9, Hnf6, Nkx6.1 and Ngn3, for example may comprise the expression of Pdx1 and Ngn3; also preferably may comprise at least the expression of Ngn3; or also preferably may comprise at least the expression of Ngn3, in conjunction with the expression of any one or more or all of Pdx1, Foxa2, Sox9, Hlxb9, Hnf6 and Nkx6.1, or in conjunction with the expression of any one or more or all of Pdx1, Foxa2, Hlxb9, Hnf6 and Nkx6.1, or in conjunction with the expression of any one or more or all of Pdx1, Hlxb9, Hnf6 and Nkx6.1, or in conjunction with the expression of any one or more or all of Hlxb9, Hnf6 and Nkx6.1. Preferably, said pancreatic endocrine progenitor cells may be negative for the expression of ALB and/or AFP, more preferably for the expression of ALB and AFP.

For example, a pancreatic endocrine cell as intended herein may comprise the ability to express a pancreatic hormone (*e.g*., insulin, glucagon, somatostatin, PP or ghrelin) normally expressed by a corresponding native, mature pancreatic endocrine cell, preferably wherein the expression of said hormone by the cell is subject to regulation which is at least qualitatively and preferably also quantitatively equivalent to the regulation observed in the corresponding native, mature pancreatic endocrine cells.

For example, a pancreatic beta-cell as intended herein may comprise the ability to express and preferably secrete insulin. More preferably, said expression and secretion of insulin may be responsive to the presence and/or level (*e.g*., concentration) of glucose in the cell or contacting the cell. Preferably, the pancreatic beta-cell may further comprise expression of any one or more or all of c-peptide, Glut-2 (Glucose transporter-2), Pdx1, PC1/3 (pro-hormone convertase 1 and 3), SUR-1 (sulfonylurea receptor-1) and synaptophysin.

The expression - for example, the presence or absence or quantity - of markers as discussed throughout this specification by cells or cell populations can be detected and/or measured using any suitable technique known in the art, such as without limitation immunological techniques including immunocytochemistry, immunofluorescence, flow cytometry and fluorescence activated cell sorting (FACS), immunoblotting including *inter alia* Western blots, dot blots and slot blots, immunoassays including *inter alia* ELISA (enzyme-linked immunosorbent assay) and RIA (radioimmunoassay) or by any suitable biochemical assay of enzyme activity, or by any suitable technique of detecting and/or measuring the marker mRNA including Northern blots, semi-quantitative or quantitative RT-PCR, array or microarray expression analysis, and so forth. Principles of the above assays are known in the art, and are further set out in the methodology guides cited elsewhere in this specification, and further *inter alia* in Ed Harlow and David Lane, "Antibodies - A Laboratory Manual", 1st ed., Cold Spring Harbor Laboratory Press 1988, ISBN 0879693142; J.M. Polak, "Introduction to Immunocytochemistry" 3rd ed., Garland Science 2003, ISBN 1859962084; M.A. Hayat, "Microscopy, Immunohistochemistry, and Antigen Retrieval Methods: For Light and Electron Microscopy", 1st ed., Springer 2002, ISBN 0306467704; John R. Crowther, "The ELISA Guidebook", 1st ed., Humana Press 2000, ISBN 0896037282; Stephen A. Bustin, "A-Z of Quantitative PCR", 1st ed., International University Line 2004, ISBN 0963681788; Anton Yuryev, "PCR Primer Design", 1st ed., Humana Press 2007, ISBN 158829725X; and others.

Sequence data including gene, transcript and protein sequence data for markers mentioned in this disclosure are generally known and can be retrieved from public databases such as for example GenBank (http://www.ncbi.nlm.nih.gov/entrez) and UniProtKB/Swiss-Prot (http://www.expasy.org). By means of example and not limitation, illustrative sequences of human markers that may be employed throughout this specification are listed here below. Each following marker entry consists of the marker name or abbreviation followed by (protein accession number GenBank, sequence version number live on February 25, 2009; accession number in UniProtKB/Swiss-Prot, sequence version number live on February 25, 2009): Bry (NP_003172, v.1; O15178, v.1), Sox17 (NP_071899, v.1; Q9H612, v.1), Gsc (NP_776248, v.1; P56915, v.2), Foxa2 (NP_068556, v.2; NP_710141, v.1; Q9Y261, v.1), CxCR4 (NP_001008540, v.1; NP_003458, v.1; P61073, v.1), Sox7 (NP_113627, v.1; Q9BT81, v.1); Pdx1 (NP_000200, v.1; P52945, v.1), Sox9 (NP_000337, v.1; P48436, v.1), Hlxb9 (NP_005506, v.3; P50219, v.2); Hnf6 (NP_004489, v.1; Q9UBC0, v.1), Nkx6.1 (NP_006159, v.2; P78426, v. 1), Ngn3 (NP_066279, v.2; Q9Y4Z2, v.1); insulin including the sequences of insulin A and B chains and C-peptide (NP_000198, v.1; P01308, v.1; these sequences are of proinsulin precursor), Glut-2 (NP_000331, v.1; P11168, v.1), synaptophysin (NP_003170, v.1; P08247, v.3).

Where a cell is said to be positive for or to express a particular marker, this means that a skilled person will conclude the presence or evidence of a distinct signal (*e.g*., antibody-detectable or detection by reverse transcription polymerase chain reaction) for that marker when carrying out the appropriate measurement compared to suitable controls (*e.g*., cells known or expected not to express the marker, *i.e.,* to be negative for said marker, *i.e.,* "negative control"). Where the method allows for quantitative assessment of the marker, positive or expressing cells may on average generate a signal that is significantly different (*e.g*., higher) from such negative control, *e.g*., but without limitation, at least 1.5-fold higher than such signal generated by the negative control, *e.g*., at least 2-fold, at least 4-fold, at least about 10-fold, at least about 20-fold, at least about 30-fold, at least about 40-fold, at least about 50-fold, at least about 100-fold, or at least about 200-fold higher or even higher.

Where a cell is said to be negative for or to not express (or substantially not express) a particular marker, this means that a skilled person will conclude the absence of a distinct signal (*e.g*., antibody-detectable or detection by reverse transcription polymerase chain reaction) for that marker when carrying out the appropriate measurement compared to suitable controls (*e.g*., cells known or expected to express the marker, *i.e.,* "positive control"; or cells known or expected not to express the marker, *i.e.,* to be negative for said marker, *i.e.,* negative control). Where the method allows for quantitative assessment of the marker, negative or non-expressing (or substantially non-expressing) cells may on average generate a signal that is comparable to or is not significantly different from such negative control, *e.g*., but without limitation, which is less than 1.5-fold of the signal generated by the control, e.g., less than 1.4-fold, less than 1.3-fold, less than 1.2-fold or less than 1.1-fold or even lower than the signal generated by the control.

The methods and protocols taught herein employ cells having definitive endoderm germ layer phenotype or cell populations comprising such cells. For example, the present methods and protocols may comprise the step (a) of providing cells having definitive endoderm germ layer phenotype. Said step (a) may as well encompass providing a cell population comprising or enriched for said cells having definitive endoderm germ layer phenotype. Such cell population may comprise, without limitation, at least about 10%, at least about 15%, at least about 20%, at least about 30%, at least about 40%, at least about 50%, at least about 60%, at least about 70%, or at least about 80% of said cells having definitive endoderm germ layer phenotype. Greater proportions of the latter cells in such cell populations are also envisaged, e.g., at least about 90%, at least about 95% or even a cell population consisting of or consisting substantially of the cells having definitive endoderm germ layer phenotype.

As used herein, the terms "definitive endoderm" and "definitive endoderm germ layer" generally refer to early endoderm cells that can under appropriate conditions give rise to any one, or preferably more, or more preferably all of endoderm cell types that are generated from the endoderm lineages during normal embryo development (in particular, pancreas, liver, lung, stomach, intestine and thyroid). Definitive endoderm cells may be typically considered multipotent (the term "multipotent" denotes the capacity of a cell to give rise to at least three cell types from each of two or more different organs or tissues of an organism, wherein the said cell types may originate from the same or from different germ layers, wherein the multipotent cell is not capable of giving rise to all of the cell types of an organism). Hence, "definitive endoderm" or "definitive endoderm germ layer" encompasses cells relatively more differentiated towards endoderm cell type(s) than a pluripotent cell from which a definitive endoderm cell may have originated (the term "pluripotent" denotes the capacity of a cell to give rise to cell types originating from all three germ layers of an organism, *i.e.,* mesoderm, endoderm, and ectoderm, and potentially capable of giving rise to any and all cell types of an organism, although not able of growing into the whole organism).

The definitive endoderm germ layer phenotype of cells may be suitably characterised or concluded by analysing the cells' expressing or not expressing certain markers, as taught in the Summary section.

For example, cells having definitive endoderm germ layer phenotype as intended herein may comprise the expression of any one or more or all of Sox17, Gsc, Foxa2 and CxCR4; or may comprise at least the expression of Sox17, preferably in conjunction with any one or more or all of Gsc, Foxa2 and CxCR4; or may comprise at least the expression of Sox17 and CxCR4 or of Sox17 and Gsc; or may comprise at least the expression of Sox17, CxCR4 and Gsc; or may comprise at least the expression of Sox17 and Foxa2, preferably in conjunction with any one or both of Gsc and CxCR4; or may preferably comprise the expression of all Sox17, Gsc, Foxa2 and CxCR4. Early during their formation from pluripotent cells, cells having definitive endoderm germ layer phenotype typically transiently express Bry.

Preferably, said cells having definitive endoderm germ layer phenotype may be negative for the expression of Sox7, and may be further negative for the expression of any one or more or preferably all of Nanog homeobox protein, AFP, SPARC (Secreted protein acidic and rich in cysteine), TM (Thrombomodulin) and Zic1 (Zic family member 1).

The production or generation of cells having definitive endoderm phenotype is known in the art and any available methods and protocols may be used herein.

By means of example and not limitation, WO 2005/063971 A2 describes a method of producing definitive endoderm phenotype cells, said method comprising the steps of : obtaining a cell population comprising pluripotent cells (*e.g*., pluripotent stem cells); providing said cell population with at least one growth factor of the TGFβ superfamily in an amount sufficient to promote differentiation of said pluripotent cells to definitive endoderm cells, said definitive endoderm cells being multipotent cells that can differentiate into cells of the gut tube or organs derived there from; and allowing sufficient time for definitive endoderm cells to form, wherein said sufficient time for definitive endoderm cells to form has been determined by detecting the presence of definitive endoderm cells in said cell population. The at least one growth factor may be of the Nodal/Activin subgroup of the TGFβ superfamily; preferably may be selected from Nodal, Activin A, Activin B and combinations thereof; said at least one growth factor may be provided in a concentration of at least about 10 ng/ml, or at least about 100 ng/ml, or at least about 500 ng/ml, or at least about 1000 ng/ml, or at least about 5000 ng/ml.

WO 2005/063971 A2 further teaches a method of producing a cell population enriched in definitive endoderm cells, said method comprising the steps of : differentiating cells in a population of pluripotent cells (*e.g*., pluripotent step cells) so as to produce definitive endoderm cells, said definitive endoderm cells being multipotent cells that can differentiate into cells of the gut tube or organs derived there from; providing to said cell population a reagent which binds to a marker expressed in said definitive endoderm cells but which is not substantially expressed in other cell types present in said cell population; and separating said definitive endoderm cells bound to said reagent from said other cell types present in said cell population, thereby producing a cell population enriched in definitive endoderm cells. Said marker may be preferably CxCR4; said reagent may be an antibody, *e.g*., an antibody having affinity for CxCR4. The methods detailed in par. [0147] to par. [0162], par. [0175] to par. [0181], par. [0209] to par. [0221], and par. [0232] to par. [0240] of WO 2005/063971 A2 are particularly relied upon, and said sections are herein incorporated by reference in their entirety.

By means of further examples and not limitation, D'Amour et al. 2005 (Nat Biotechnol 23: 1534-41) and D'Amour *et al.* 2006 (*supra*) describe methods of producing definitive endoderm phenotype cells, said method comprising exposing pluripotent (ES) cells to relatively high concentrations of Activin A (100 ng/ml) in the context of no or low FBS supplementation (0% and 0.2%), preferably for between 2 and 4 days and also preferably adding Wnt3a during the first 1 or 2 days of Activin A exposure.

Thus, in an embodiment, the cells having definitive endoderm germ layer phenotype as provided in step (a) of the present methods may be obtainable or directly obtained by *in vitro* method comprising the steps of: (i) providing pluripotent stem cells, preferably mPS cells, more preferably hPS cells; and (ii) differentiating said pluripotent stem cells, mPS cells or hPS cells into cells having definitive endoderm germ layer phenotype.

Said step (ii) may comprise exposing said pluripotent stem cells, mPS cells or hPS cells to one or more growth factors or ligands of the TGFβ superfamily in no-serum (*i.e.,* serum-free) conditions or low-serum conditions (e.g., serum 0.5% vol/vol or lower, preferably about 0.4% vol/vol or lower, more preferably about 0.3% vol/vol or lower, yet more preferably about 0.2% vol/vol).

The duration of step (ii) may be generally chosen to be adequate to allow for the definitive endoderm cells to form. Said adequate duration may be determined by detecting the presence (*e.g*., by detecting the expression of corresponding markers) of definitive endoderm cells in the resulting cell population. By means of example, an adequate duration may be concluded when the resulting cell population comprises at least about 5%, at least about 10%, preferably at least about 15%, more preferably at least about 20% and even more preferably at least about 30%, even more preferably at least about 40%, at least about 50%, more preferably at least about 60%, yet more preferably at least about 70%, still more preferably at least about 80% or more of definitive endoderm phenotype cells.

In an embodiment, the duration of said step (ii) may be between about 2 days and about 6 days, preferably between about 3 days and about 5 days.

In an embodiment, said step (ii) may comprise (ii a) exposing said pluripotent stem cells, mPS cells or hPS cells to one or more growth factors or ligands of the TGFβ superfamily and to one or more Wnt ligands, or BMP ligands or sodium butyrate, preferably to one or more Wnt ligands, in no-serum conditions or low-serum conditions as defined above, and (ii b) subsequently exposing said cells to one or more growth factors or ligands of the TGFβ superfamily in no-serum conditions or low-serum conditions as defined above.

In an embodiment, said step (ii) may comprise (ii a) exposing said pluripotent stem cells, mPS cells or hPS cells to one or more growth factors or ligands of the TGFβ superfamily and to one or more Wnt ligands, or BMP ligands or sodium butyrate, preferably to one or more Wnt ligands, in no-serum conditions, and (ii b) subsequently exposing said cells to one or more growth factors or ligands of the TGFβ superfamily in low-serum conditions as defined above.

Preferably, said TGFβ factor or ligand may be of the Nodal/Activin subgroup of the TGFβ superfamily, more preferably may be Nodal, Activin A, or Activin B, even more preferably may be Activin A. Exemplary but non-limiting concentration of the TGFβ factor or ligand such as Activin A may be at least about 30 ng/ml, preferably at least about 50 ng/ml, more preferably at least about 80 ng/ml, even more preferably at least about 100 ng/ml, typically about 100 ng/ml.

Preferably, said Wnt ligand may be Wnt3a. Exemplary but non-limiting concentration of the Wnt ligand such as Wnt3a may be at least about 10 ng/ml, preferably at least about 20 ng/ml, more preferably at least about 25 ng/ml, typically about 25 ng/ml.

In an embodiment, the duration of said step (ii a) may be between 1 and 3 days, more preferably 1 or 2 days, even more preferably 1 day. In an embodiment, the duration of said step (ii b) may be between 2 and 4 days, more preferably 2 or 3 days, even more preferably 2 days.

Said step (i) may as well encompass providing a cell population comprising or enriched for said pluripotent stem cells, mPS cells or hPS cells. Such cell population may comprise, without limitation, at least about 50%, preferably at least about 60%, more preferably at least about 70%, even more preferably at least about 80%, yet more preferably or at least about 90% or at least about 95% of said pluripotent stem cells, mPS cells or hPS cells. A cell population consisting of or consisting substantially of pluripotent stem cells, mPS cells or hPS cells is also envisaged.

In a preferred embodiment, at the outset (start) of said step (ii) the pluripotent stem cells, mPS cells or hPS cells or culture comprising such as provided in said step (i) may be at least about 50% confluent, more preferably at least about 60% confluent, even more preferably at least about 70% confluent, yet more preferably at least about 80% confluent, such as, for example, between about 60% and about 90% confluent, more preferably between about 70% and about 90% confluent, still more preferably between about 75% and about 85% confluent, yet more preferably about 80% confluent. The term "confluence" generally denotes a density of cultured cells in which the cells contact one another covering substantially all of the surfaces available for growth (*i.e.,* fully or 100% confluent).

As explained above, cells having definitive endoderm germ layer phenotype may be suitably prepared *inter alia* from pluripotent stem cells, such as mPS or hPS.

The term "stem cell" generally refers to a progenitor cell capable of self-renewal, *i.e.,* which can under appropriate conditions proliferate without differentiation. The term encompasses stem cells capable of substantially unlimited self-renewal, *i.e.,* wherein at least a portion of the stem cell's progeny substantially retains the unspecialised or relatively less specialised phenotype, the differentiation potential, and the proliferation capacity of the mother stem cell; as well as stem cells which display limited self-renewal, *i.e.,* wherein the capacity of the stem cell's progeny for further proliferation and/or differentiation is demonstrably reduced compared to the mother cell.

The term "mammalian pluripotent stem cell" or "mPS" cell generally refers to a pluripotent stem cell of mammalian origin. In an embodiment, the mPS cells may be derived from a non-human mammal. For example, in an embodiment the mPS cells may be derived from a laboratory animal, e.g., laboratory mammal, preferably from mouse, rat, hamster or rabbit, more preferably mouse. In another preferred embodiment, the mPS cells may be derived from pig. In yet another preferred embodiment, the mPS cells may be derived from primate, such as from non-human primate. In yet another preferred embodiment, the mPS cells may be derived from human (hPS cells).

Prototype mPS cell is a pluripotent stem cell derived from any kind of mammalian embryonic tissue, *e.g*., embryonic, foetal or pre-foetal tissue, the cell being capable under appropriate conditions of producing progeny of different cell types that are derivatives of all three germ layers, *i.e.,* endoderm, mesoderm, and ectoderm, according to a standard art-accepted test, such as *inter alia* the ability to form a teratoma in SCID mice, or the ability to form identifiable cells of all three germ layers in tissue culture.

Included in the definition of mPS cells are without limitation embryonic stem cells of various types, exemplified without limitation by murine embryonic stem cells, e.g., as described by Evans & Kaufman 1981 (Nature 292: 154-6) and Martin 1981 (PNAS 78: 7634-8); rat pluripotent stem cells, *e.g*., as described by lannaccone et al. 1994 (Dev Biol 163: 288-292); hamster embryonic stem cells, *e.g*., as described by Doetschman et al. 1988 (Dev Biol 127: 224-227); rabbit embryonic stem cells, *e.g*., as described by Graves et al. 1993 (Mol Reprod Dev 36: 424-433); porcine pluripotent stem cells, *e.g*., as described by Notarianni et al. 1991 (J Reprod Fertil Suppl 43: 255-60) and Wheeler 1994 (Reprod Fertil Dev 6: 563-8); sheep embryonic stem cells, *e.g*., as described by Notarianni *et al.* 1991 (*supra*); bovine embryonic stem cells, *e.g*., as described by Roach et al. 2006 (Methods Enzymol 418: 21-37); human embryonic stem (hES) cells, *e.g*., as described by Thomson et al. 1998 (Science 282: 1145-1147); human embryonic germ (hEG) cells, e.g., as described by Shamblott et al. 1998 (PNAS 95: 13726); embryonic stem cells from other primates such as Rhesus stem cells, *e.g*., as described by Thomson et al. 1995 (PNAS 92:7844-7848) or marmoset stem cells, *e.g*., as described by Thomson et al. 1996 (Biol Reprod 55: 254-259).

Other types of mPS cells are also included in the term as are any cells of mammalian origin capable of producing progeny that includes derivatives of all three germ layers, regardless of whether they were derived from embryonic tissue, foetal tissue or other sources. mPS cells are preferably not derived from a malignant source. A cell or cell line is from a "non-malignant source" if it was established from primary tissue that is not cancerous, nor altered with a known oncogene. It may be desirable that the mPS maintain a normal karyotype throughout prolonged culture under appropriate conditions. It may also be desirable, but not always necessary, that the mPS maintain substantially indefinite self-renewal potential under appropriate *in vitro* conditions.

As noted, prototype "human ES cells" are described by Thomson *et al.* 1998 (*supra*) and in US 6,200,806. The scope of the term covers pluripotent stem cells that are derived from a human embryo at the blastocyst stage, or before substantial differentiation of the cells into the three germ layers. ES cells, in particular hES cells, are typically derived from the inner cell mass of blastocysts or from whole blastocysts. Derivation of hES cell lines from the morula stage has been documented and ES cells so obtained can also be used in the invention (Strelchenko et al. 2004. Reproductive BioMedicine Online 9: 623-629). As noted, prototype "human EG cells" are described by Shamblott *et al.* 1998 (*supra*). Such cells may be derived, *e.g*., from gonadal ridges and mesenteries containing primordial germ cells from foetuses. In humans, the foetuses may be typically 5-11 weeks post-fertilisation.

Those skilled in the art will appreciate that, except where explicitly required otherwise, the term mPS cells may include primary tissue cells and established lines that bear phenotypic characteristics of the respective cells, and derivatives of such primary cells or cell lines that still have the capacity of producing progeny of each of the three germ layers.

Exemplary but non-limiting established lines of human ES cells include lines which are listed in the NIH Human Embryonic Stem Cell Registry (http://stemcells.nih.gov/research/registry), and sub-lines thereof, such as, lines hESBGN-01, hESBGN-02, hESBGN-03 and hESBGN-04 from Bresagen Inc. (Athens, GA), lines Sahlgrenska 1 and Sahlgrenska 2 from Cellartis AB (Göteborg, Sweden), lines HES-1, HES-2, HES-3, HES-4, HES-5 and HES-6 from ES Cell International (Singapore), line Miz-hES1 from MizMedi Hospital (Seoul, Korea), lines I 3, I 3.2, I 3.3, I 4, I 6, I 6.2, J 3 and J 3.2 from Technion - Israel Institute of Technology (Haifa, Israel), lines HSF-1 and HSF-6 from University of California (San Francisco, CA), lines H1, H7, H9, H13, H14 of Wisconsin Alumni Research Foundation / WiCell Research Institute (Madison, WI), lines CHA-hES-1 and CHA-hES-2 from Cell & Gene Therapy Research Institute / Pochon CHA University College of Medicine (Seoul, Korea), lines H1, H7, H9, H13, H14, H9.1 and H9.2 from Geron Corporation (Menlo Park, CA), lines Sahlgrenska 4 to Sahlgrenska 19 from Göteborg University (Göteborg, Sweden), lines MB01, MB02, MB03 from Maria Biotech Co. Ltd. (Seoul, Korea), lines FCNCBS1, FCNCBS2 and FCNCBS3 from National Centre for Biological Sciences (Bangalore, India), and lines RLS ES 05, RLS ES 07, RLS ES 10, RLS ES 13, RLS ES 15, RLS ES 20 and RLS ES 21 of Reliance Life Sciences (Mumbai, India). Other exemplary established hES cell lines include those deposited at the UK Stem Cell Bank (http://www.ukstemcellbank.org.uk/), and sub-lines thereof, e.g., line WT3 from King's College London (London, UK) and line hES-NCL1 from University of Newcastle (Newcastle, UK) (Strojkovic et al. 2004. Stem Cells 22: 790-7). Further exemplary ES cell lines include lines FC018, AS034, AS034.1, AS038, SA111, SA121, SA142, SA167, SA181, SA191, SA196, SA203 and SA204, and sub-lines thereof, from Cellartis AB (Göteborg, Sweden).

In a preferred embodiment, human ES cell lines suitable for the present invention may include the ES cell lines VUB07, VUB02, VUB01 and VUB14 established as described in Mateizel et al. 2006. (Hum. Reprod 21: 503-511) and deposited (VUB01, VUB02, and VUB07) at the European Human Embryonic Stem Cell Registry (www.hESCreg.eu; Borstlap et al. 2008. Regen Med. 3(6): 945-51).

Further within the term mammalian pluripotent stem cells are such mPS cells obtainable by manipulation, such as *inter alia* genetic and/or growth factor mediated manipulation, of non-pluripotent mammalian cells, such as somatic and particularly adult somatic mammalian cells, including the use of induced pluripotent stem (iPS) cells, as taught *inter alia* by Yamanaka et al. 2006 (Cell 126: 663-676) and Yamanaka et al. 2007 (Cell 131: 861-872).

A skilled person will appreciate that further cell lines having characteristics of mammalian, esp. mouse or human, pluripotent cells, esp. ES cells or EG cells, may be established in the future, and these may too be suitable in the present invention. A skilled person can also use techniques known in the art to verify that any established or yet to be established mPS cell lines, or sub-lines thereof, show desirable cell characteristics, such as expansion *in vitro* in undifferentiated state, preferably normal karyotype and ability of pluripotent differentiation.

The present methods and protocols may preferably depart from pluripotent stem cell populations (*e.g*., mPS or hPS cell populations) which are "undifferentiated", *i.e.,* wherein a substantial proportion (for example, at least about 60%, preferably at least about 70%, even more preferably at least about 80%, still more preferably at least about 90% and up to 100%) of cells in the stem cell population display characteristics (*e.g*., morphological features and/or markers) of undifferentiated mPS cells, clearly distinguishing them from cells undergoing differentiation.

Undifferentiated mPS cells are generally easily recognised by those skilled in the art, and may appear in the two dimensions of a microscopic view with high nuclear/cytoplasmic ratios and prominent nucleoli, may grow as compact colonies with sharp borders. It is understood that colonies of undifferentiated cells within the population may often be surrounded by neighbouring cells that are more differentiated. Nevertheless, the undifferentiated colonies persist when the population is cultured or passaged under appropriate conditions known *per se,* and individual undifferentiated cells constitute a substantial proportion of the cell population. Undifferentiated mPS cells may express the stage-specific embryonic antigens (SSEA) 3 and 4, and markers detectable using antibodies designated Tra-1-60 and Tra-1-81 (Thomson *et al.* 1998, *supra*).

Undifferentiated mPS cells may also typically express Nanog, Oct-4 and TERT. Undifferentiated mPS cells may also comprise expression of alkaline phosphatase (AP) (e.g., as determined by a suitable AP activity assay).

Culture conditions which allow for continuous proliferation of pluripotent stem cells, mPS or hPS cells in culture substantially without inducing differentiation are generally known in the art.

By means of example and not limitation, serum-containing hES cell medium may be made with 80% DMEM (such as Knock-Out DMEM, Gibco), 20% of either defined foetal bovine serum (FBS, Hyclone) or serum replacement (*e.g*., WO 98/30679, *i.e.,* serum-free conditions), 1% non-essential amino acids,1 mM L-glutamine, and 0.1 mM β-mercaptoethanol. Just before use, human b-FGF may be added to 4ng/mL (WO 99/20741).

Conventionally, albeit in no way limiting, ES cells may be cultured on a layer of feeder cells, typically fibroblasts derived from embryonic or foetal tissue, typically mouse tissue. In a non-limiting example, embryos may be harvested from a CF1 mouse at 13 days of pregnancy, transferred to 15 mL trypsin/EDTA, finely minced, and incubated 25 min at 37°C. Medium comprising 90% DMEM, 10% FBS, 1% non essential amino acids and 2 mM glutamine was added, the debris is allowed to settle, and the cells are propagated in the same medium. To prepare a feeder cell layer, cells are treated to inhibit proliferation but permit synthesis of factors that support ES cells. Culture plates are coated with 0.1% gelatine for 2 h, plated with 375,000 mitomycine-treated (10 µg/ml) mEF per well, and used up to 4 days after plating. The medium is replaced with fresh hES cell medium just before seeding the pluripotent stem cells, mPS or hPS.

Alternatively, pluripotent stem cells, mPS or hPS cells may be cultured on mammalian, e.g., primate or human fibroblasts. For example, Genbacev et al. 2005 (Fertil Steril. 83: 1517-29) described culturing of hES cells on pathogen-free human placental fibroblast feeders, and in serum-free conditions, Lee et al. 2005 (Biol Reprod 72: 42-9) and Lee et al. 2004 (Reproduction 128: 727-35) disclosed culturing of hES cells on mitotically inactivated human uterine endometrial cells, and Richards et al. 2002 (Nat Biotechnol 20: 933-6) described culturing of hES cells on human foetal and adult fibroblast feeders. Furthermore, pluripotent stem cells, mPS or hPS cells may be propagated in the absence of a feeder cell layer. For example, Xu et al. 2001 (Nature Biotechnology 19: 971) discloses culturing of pluripotent stem cells in the absence of feeder cells, wherein the cells are cultured on an extracellular matrix in a medium conditioned by MEF cells, WO 2001/51616 discloses culturing of pluripotent stem cells in the absence of feeder cells, wherein the cells are cultured on an extracellular matrix, e.g., Matrigel ® Basement Membrane Matrix (*e.g*., BD Biosciences) or laminin, and in a medium conditioned by primary or permanent cell lines, *e.g*., primary embryonic fibroblasts, telomerised fibroblasts, and fibroblast cells differentiated and selected from cultured pluripotent stem cells, and WO 2003/020920 and WO 2006/017370 disclose feeder-free culturing of pluripotent stem cells on support structures, such as extracellular matrix, in media supplemented with sufficient fibroblast growth factor.

The present methods and protocols generally involve exposing cells or cell populations to one or more of a variety of agents (*e.g*., antagonists, agonists, ligands, etc. of certain signalling pathways or signalling processes or molecules) or conditions.

The terms "expose" or "exposing" generally denote contacting or bringing together, either directly or indirectly, that which is being "exposed" with one or more agents or conditions (e.g., one or more molecules, components, materials or conditions), thereby facilitating interactions there between. Typically, cells or a cell population may be exposed to an agent or condition by means of including said agent or condition in culture media in which said cells or cell population are cultured.

The term "antagonist" or "inhibitor" of a pathway or signalling refers to any substance, molecule or macromolecule, such as a natural, semi-synthetic, synthetic or recombinant substance, molecule or macromolecule, that decreases, inhibits or abolishes the activity of said pathway or signalling, such as for example directly by affecting a component (*e.g*., protein, receptor, ligand, transcription factor, growth factor, hormone, *etc*.) involved in said pathway or signalling or by affecting upstream or downstream mediators of said pathway or signalling.

The term "agonist" or "activator" of a pathway or signalling refers to any substance, molecule or macromolecule, such as a natural, semi-synthetic, synthetic or recombinant substance, molecule or macromolecule, that induces, increases or stimulates the activity of said pathway or signalling, such as for example directly by affecting a component (*e.g*., protein, receptor, ligand, transcription factor, growth factor, hormone, *etc*.) involved in said pathway or signalling or by affecting upstream or downstream mediators of said pathway or signalling.

The term "ligand" generally refers to a substance, molecule or macromolecule capable of binding (*i.e.,* capable of a physical association or interaction, preferably a non-covalent physical association or interaction) with a recited molecular entity, such as with a receptor, e.g., with a receptor (*e.g*., peptide, polypeptide or protein) participating in a pathway or signalling.

A ligand may bind "specifically" with its cognate receptor(s), *e.g*., with the native conformation of said receptor. "Specific binding" generally means that a ligand binds to its cognate receptor substantially to the exclusion of other molecules which are random or unrelated, and optionally substantially to the exclusion of other molecules that are structurally related. For example, the affinity of a ligand for binding with its cognate receptor(s) under the conditions of binding may be at least about 2-fold greater, preferably at least about 5-fold greater, more preferably at least about 10-fold greater, yet more preferably at least about 25-fold greater, still more preferably at least about 50-fold greater, and even more preferably at least about 100-fold or more greater, than its affinity for a non-target molecule. Preferably, a ligand may bind to its cognate receptor(s) with affinity constant (K_{A}) of such binding K_{A} ≥ 1×10⁶ M⁻¹, more preferably K_{A} ≥ 1×10⁷ M⁻¹, yet more preferably K_{A} ≥ 1×10⁸ M⁻¹, even more preferably K_{A} ≥ 1×10⁹ M⁻¹, and still more preferably K_{A} ≥ 1×10¹⁰ M⁻¹ or K_{A} ≥ 1×10¹¹ M⁻¹, wherein K_{A} = [Ligand_Receptor]/[Ligand][Receptor]. Determination of K_{A} can be carried out by methods known in the art, such as for example, using equilibrium dialysis and Scatchard plot analysis.

Upon binding thereto, a ligand may qualitatively and/or quantitatively affect the activity of its cognate receptor(s). For example, the ligand may decrease, inhibit or abolish the activity of said receptor(s). Alternatively, the ligand may induce, increase or stimulate the activity of said receptor(s). As intended herein, a ligand of given receptor(s) (e.g., a peptide, polypeptide or protein) may preferably induce, increase or stimulate the activity of said receptor(s).

Without limitation, antagonists, agonists or ligands as intended herein may take form of a peptide, a polypeptide, a protein, a chemical or biological substance (e.g., a small organic molecule activator or inhibitor, preferably having size up to about 5000 Da, e.g., up to about 4000, preferably up to about 3000 Da, more preferably up to 2000 Da, even more preferably up to about 1000 Da, e.g., up to about 900, 800, 700, 600 or up to about 500 Da), a pharmaceutical agent or drug, a specific binding agent such as a neutralising or antagonistic antibody, an antibody, a peptide, a peptidomimetic, an aptamer, a nucleic acid molecule or oligonucleotide, or a dominant negative fragment or variant of a protein or polypeptide involved in a pathway or signalling.

In an embodiment, a pathway or signalling may be antagonised by reducing the expression of one or more molecules such as proteins or polypeptides involved therein that normally activate or stimulate said pathway or signalling. Alternatively, a pathway or signalling may be agonised by reducing the expression of one or more molecules such as proteins or polypeptides involved therein that normally inhibit or reduce said pathway or signalling. This may encompass any extent of reduction of expression, such as, e.g., by ≥10%, *e.g.,* ≥20%, more preferably ≥30%, *e.g.,* ≥40%, yet more preferably ≥50%, *e.g.,* ≥60%, still more preferably ≥70%, e.g., ≥80%, and most preferably by ≥90%, e.g., ≥95% or even about 100%, relative to the basal expression level of the respective protein(s); as determined using quantification methods known *per se,* such as, e.g., ELISA, RIA, immuno-precipitation, Western blotting, etc. Decreasing the expression level of a desired gene product may be achieved by methods known in the art, such as, e.g., by transfecting (e.g., by electroporation, lipofection, etc.) or transducing (e.g., using a viral vector), the cell with an antisense agent, such as, e.g., antisense DNA or RNA oligonucleotide, a construct encoding for an antisense transcript, or an RNAi agent, such as siRNA, shRNA or vectors encoding such, etc.

In an embodiment, a pathway or signalling may be agonised by increasing the expression of one or more molecules such as proteins or polypeptides involved therein that normally activate or stimulate said pathway or signalling. Alternatively, a pathway or signalling may be antagonised by increasing the expression of one or more molecules such as proteins or polypeptides involved therein that normally inhibit or reduce said pathway or signalling. An increase in the expression level of a protein in a cell may be achieved by methods known in the art, such as, *e.g*., by transfecting (*e.g*., by electroporation, lipofection, etc.) or transducing (e.g., using a viral vector), the cell with a recombinant nucleic acid which encodes said protein under the control of a promoter effecting suitable expression level in said cell.

In an embodiment, a pathway or signalling may be antagonised or agonised by, respectively, reducing or increasing the activity and/or availability of one or more molecules such as proteins or polypeptides involved in said a pathway or signalling, wherein said molecules normally activate or stimulate said pathway or signalling; or by, respectively, reducing or increasing binding between two or more molecules such as proteins or polypeptides involved in said a pathway or signalling, wherein said binding normally activates or stimulates said pathway or signalling.

In an embodiment, a pathway or signalling may be agonised or antagonised by, respectively, reducing or increasing the activity and/or availability of one or more molecules such as proteins or polypeptides involved in said a pathway or signalling, wherein said molecules normally inhibit or reduce said pathway or signalling; or by, respectively, reducing or increasing binding between two or more molecules such as proteins or polypeptides involved in said a pathway or signalling, wherein said binding normally inhibits or reduces said pathway or signalling.

Inhibition or antagonism of the BMP pathway as intended herein may be suitably determined and/or quantitated by measuring the phosphorylation status of BMP-specific SMAD1/5/8 proteins as described *inter alia* in Funaba et al. 2007 (J Biochem. Biophys. Methods 70:816-819).

In an embodiment, an antagonist of BMP pathway may affect the activity and/or availability of any one or more components or members of the BMP signalling pathway, such as a component or member chosen from the group comprising or consisting of BMP1-BMP7, BMP8a, BMP8b, BMP10 and BMP15, or a ligand or a receptor thereof or a native interaction partner thereof. Preferably, the antagonist may affect the activity and/or availability of BMP2 and BMP4.

In an embodiment, the antagonist of BMP pathway may be chosen from the group comprising or consisting of noggin, chordin, grhemlin, sclerostin, Cerberus, GDF3 (growth/differentiation factor-3), Dan, Coco, PRDC, short gastrulation and twisted gastrulation.

Without limitation, noggin may be used at an effective concentration between about 50 and about 200 ng/ml, preferably at about 100 ng/ml. It may be regularly refreshed in the medium of cells, such as without limitation every other day.

Activation of FGF signalling as intended herein may be suitably determined and/or quantitated by measuring the phosphorylation status of ERK1/2 (extracellular-regulated kinase-1/-2) and PKB (protein kinase B) or Akt as described *inter alia* in Paling et al. 2004 (J. Biol. Chem. 279: 48063-48070) and in Li et al. 2007 (Differentiation 75: 299-307).

In an embodiment, an activator or agonist of FGF signalling may affect the activity and/or availability of any one or more components or members of the FGF signalling, such as a component or member chosen from the group comprising or consisting of FGF 1 (acidic), FGF 2 (basic), FGF 3-23, or a ligand or a receptor thereof or a native interaction partner thereof. Preferably, the agonist may affect the activity and/or availability of FGF10, FGF7 and FGF2.

In an embodiment, the agonist of FGF signalling may be a growth factor selected from the FGF family, more preferably FGF-10.

Without limitation, FGF10 may be used at an effective concentration between about 10 and about 100 ng/ml, preferably at about 50 ng/ml. It may be regularly refreshed in the medium of cells, such as without limitation every other day.

Inhibition or antagonism of the hedgehog pathway as intended herein may be suitably determined and/or quantitated by measuring the level of downstream effectors. For example, inhibition of sonic hedgehog signalling may be suitably determined and/or quantitated by measuring the expression level of Gli1 transcripts (e.g., by RTPCR or any other transcript detection method) as described in Mfopou et al. 2007 (Stem Cells 25:1156-1165).

In an embodiment, an antagonist of hedgehog pathway signalling may affect the activity and/or availability of any one or more components or members of the hedgehog signalling, such as a component or member chosen from the group comprising or consisting of Desert Hedgehog (Dhh), Indian Hedgehog (Ihh), and Sonic Hedgehog (Shh), or a ligand or a receptor thereof (such as, e.g., Patched (Ptc), and Smo (Smoothened)) or a native interaction partner thereof (such as, e.g., glioma-associated oncogene homolog (Gli)). Preferably, the antagonist may affect the activity and/or availability of Smo and result in the unavailability of Gli1.

In an embodiment, an antagonist of hedgehog pathway may be chosen from the group comprising or consisting of: cyclopamine (Incardona et al. 1998. Development 125: 3553-3562), cyclopamine analogues (such as, *e.g*., disclosed in US 2006/074030), KAAD-cyclopamine (3-Keto-N-(aminoethyl-aminocaproyl-dihydrocinnamoyl)cyclopamine), tomatidine, jervine, cholesterol, tripanarol, AY9944, the pyridyl inhibitors disclosed in WO 2006/028958, anti-hedgehog antibodies such as 5E1 (Erickson et al. 1996. Cell 87: 661-73), hedgehog interacting proteins (Hhip), and small molecule inhibitors such as Cur61414 (Williams et al. 2003. PNAS 100: 4616-4621).

Without limitation, KAAD-cyclopamine may be used at an effective concentration between about 100 and about 500 nM, preferably at about 250 nM. It may be regularly refreshed in the medium of cells, such as without limitation every other day.

Activation of retinoid signalling as intended herein may be suitably determined and/or quantitated using a DR5-RARE (retinoic acid receptor responsive element) transgene reporter as described *inter alia* in Thompson Haskell et al. 2002 (J. Comp. Neurol. 452: 223-241).

In an embodiment, an activator or agonist of retinoid signalling may affect the activity and/or availability of any one or more components or members of the retinoid signalling, such as a component or member chosen from the group comprising or consisting of retinoic acid intracellular receptors, such as for example retinoic acid receptor alpha (RARA), retinoic acid receptor beta (RARB) and retinoic acid receptor gamma (RARG).

In an embodiment, the activator of retinoid signalling is chosen from the group comprising or consisting of retinoic acid, retinol and retinal.

Without limitation, all-trans retinoic acid may be used at an effective concentration between about 0.5 and about 5 µM, preferably at about 2 µM. It may be regularly refreshed in the medium of cells, such as without limitation every other day.

Inhibition or antagonism of the Notch pathway as intended herein may be suitably determined and/or quantitated by measuring the transcripts and protein levels of Hes1 and Hes5, the downstream effectors of the Notch pathway (Ohtsuka et al. 1999, The EMBO Journal 18: 2196-2207) or by measuring the residual gamma secretase activity (RnD Systems, Gamma-secretase activity kit, FP003) in cell lysates upon treatment with a gamma secretase inhibitor.

In an embodiment, an antagonist of Notch pathway signalling may affect the activity and/or availability of any one or more components or members of the Notch signalling, such as a component or member chosen from the group comprising or consisting of Notch1, Notch2 or Notch3, or a ligand or a receptor thereof or a native interaction partner thereof. Preferably, the antagonist may affect the activity and/or availability of Notch1.

In an embodiment, the antagonist of Notch pathway is chosen from the group comprising or consisting of gamma secretase/presenilin inhibitors, recombinant notch extracellular domain, activators of notch intracellular domain degradation (SEL-10), and antibodies to notch receptor ligands including delta, serrate, jagged.

"Gamma secretase inhibitor" generally refers to any molecule that inhibits gamma secretase or signaling events caused by the activity of gamma secretase. Exemplary gamma secretase inhibitors include, but are not limited to, N-[N-(3,5- Diflurophenacetyl-L-alanyl)]-S-phenylglycine t-Butyl Ester (DAPT), the F-box protein SEL-10, Compound E ([(2S)-2-{[(3,5-Difluorophenyl)acetyl]amino}-*N*-[(3S)-1-methyl-2-oxo-5-phenyl-2,3-dihydro-1*H*-1,4-benzodiazepin-3-yl]propanamide]) (Seiffert et al. 2000. J Biol Chem 275: 34086), gamma secretase blocking antibodies and any other inhibitors of gamma secretase function known to those of ordinary skill in the art.

Without limitation, compound E may be used at an effective concentration between about 0.1 and about 10 µM, preferably at about 1 µM. It may be regularly refreshed in the medium of cells, such as without limitation every other day.

Activation of glucagon-like peptide 1 (Glp-1) receptor as intended herein may be suitably determined and/or quantitated by measuring the phosphorylation status of downstream effectors such as extracellular-regulated kinase (ERK1/2), protein kinase A (PKA) and/or phosphoinositide-3 kinase (PI3K), though these are not specific to the pathway.

In an embodiment, an activator or agonist of Glp-1 receptor may be chosen from the group comprising or consisting of exendin-4.

Without limitation, exendin-4 may be used at an effective concentration between about 10 and about 100 ng/ml, preferably at about 50 ng/ml. It may be regularly refreshed in the medium of cells, such as without limitation every other day.

Inhibition or antagonism of the FGF signalling as intended herein may be suitably determined and/or quantitated by measuring the phosphorylation status of ERK1/2.

In an embodiment, an antagonist of FGF signalling may negatively affect the activity and/or availability of any one or more components or members of the FGF signalling, such as a component or member chosen from the group comprising or consisting of FGF 1 (acidic), FGF 2 (basic), FGF 3-23, or a ligand or receptor thereof or a native interaction partner thereof. Preferably, the antagonist may negatively affect the activity and/or availability of FGF receptors, such as, e.g., FGFR1 and FGFR2.

It is generally intended herein that the recited agents and conditions (*e.g*., agonists, antagonists, ligands, *etc*.) are provided in addition to, *i.e.,* exogenously to or in supplement to, such agents and conditions that may be endogenously produced by the cultured cells or cell populations. Also, the recited agents or conditions may be provided exogenously to such agents and conditions that may already be preset in the medium, such as for example by means of a medium supplement, for example a complex undefined supplement, such as in particular in serum or plasma.

Where a reference is made herein to certain peptides, polypeptides or proteins (such as, *e.g*., for use as agents herein), such peptides, polypeptides or proteins may be preferably of animal origin, more preferably of mammalian origin, such as of non-human mammalian or human origin, preferably may be of same origin as the cells being treated. Said peptides, polypeptides or proteins may be, e.g., isolated from biological sources, produced by recombinant means, or produced by synthetic means.

Where a reference is made herein to certain peptides, polypeptides or proteins (such as, e.g., for use as agents herein), such reference is to be understood as also encompassing functional fragments and/or variants of said peptides, polypeptides or proteins.

The term "fragment" generally denotes a N- and/or C-terminally truncated form of a peptide, polypeptide or proteins. Preferably, a fragment may comprise at least about 30%, *e.g*., at least 50% or at least 70%, preferably at least 80%, *e.g*., at least 85%, more preferably at least 90%, and yet more preferably at least 95% or even about 99% of the amino acid sequence length of said peptide, polypeptide or protein.

The term "variant" of a recited given peptide, polypeptide or protein to peptides, polypeptides or proteins the amino acid sequence of which is substantially identical (*i.e.,* largely but not wholly identical) to the sequence of said recited peptide, polypeptide or protein, e.g., at least about 85% identical, *e.g*., preferably at least about 90% identical, *e.g*., at least 91% identical, 92% identical, more preferably at least about 93% identical, *e.g*., 94% identical, even more preferably at least about 95% identical, *e.g*., at least 96% identical, yet more preferably at least about 97% identical, *e.g*., at least 98% identical, and most preferably at least 99% identical.

Sequence identity may be determined using suitable algorithms for performing sequence alignments and determination of sequence identity as know per se. Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, *e.g*., for the BLASTN algorithm: cost to open a gap = 5, cost to extend a gap = 2, penalty for a mismatch = -2, reward for a match = 1, gap x_dropoff = 50, expectation value = 10.0, word size = 28; or for the BLASTP algorithm: matrix = Blosum62, cost to open a gap = 11, cost to extend a gap = 1, expectation value = 10.0, word size = 3).

The term "functional" denotes that fragments and/or variants at least partly retain the biological activity or functionality of the recited peptides, polypeptides or proteins. Preferably, such functional fragments and/or variants may retain at least about 20%, *e.g*., at least 30%, or at least 40%, or at least 50%, e.g., at least 60%, more preferably at least 70%, *e.g*., at least 80%, yet more preferably at least 85%, still more preferably at least 90%, and most preferably at least 95% or even 100% or higher of the activity (such as, *e.g*., ability to induce or inhibit a pathway or signalling) of the corresponding peptides, polypeptides or proteins.

Where a reference is made herein to certain substances or molecules or biological molecules such as peptides, polypeptides or proteins (such as, e.g., for use as agents herein), such reference is to be understood as also encompassing functional (*i.e.,* adequately achieving the desired effect or function) derivatives and analogues of substances or molecules or biological molecules. For example, such derivatives or analogues may encompass chemical modifications (*e.g*., additions, omissions or substitutions of atoms and/or moieties) , and/or biological modifications (*e.g*., post-expression modifications including, for example, phosphorylation, glycosylation, lipidation, methylation, cysteinylation, sulphonation, glutathionylation, acetylation, oxidation of methionine to methionine sulphoxide or methionine sulphone, and the like).

Also described herein are *in vitro* methods and protocols whereby the pancreatic progenitor cells or cell populations, or preferably the pancreatic endocrine progenitor cells or cell populations, are further differentiated into pancreatic endocrine cells, such as beta-cells.

Whereas agents and conditions for inducing such differentiation may be further identified and optimised also using the present progenitor cells and cell populations, exemplary agents to which said progenitor cells and cell populations may be exposed to induce differentiation to beta-cells include without limitation nicotinamide, glucagon-like peptide 1 and its agonists such as, e.g. Exendin-4, vascular endothelial growth factor (VEGF), FGF2 (basic), bone morphogenetic protein 4 (BMP4), betacellulin, activin A and B, conophylline, Indian hedgehog and PI3K inhibitors. Exemplary methods and protocols for such later or final stages of differentiation to beta-cells are described without limitation in Zhang et al. 2009 (Cell Research advance online publication 3 march 2009, doi: 10.1038/cr.2009.28), Jiang et al. 2007 (Stem Cells 25: 1940-1953), D'Amour et al. 2006 (Nat. Biotechnol. 24: 1392-1401).

Also disclosed herein are isolated cells and cell populations, in particular isolated pancreatic progenitor cells, pancreatic endocrine progenitor cells and pancreatic endocrine cells (for example beta-cells) and isolated cell populations comprising any one or more of said cell types. Said cell types and cell populations taught herein may be characterised by the respective properties as disclosed herein (such as, for example, may comprise the respective marker expression and/or other phenotypic properties taught herein) and/or may be obtainable or directly obtained by the herein disclosed methods.

Further contemplated are the progeny of the herein taught cell types and cell populations, including relatively more differentiated progeny, in particular mature pancreatic endocrine cells (for example, beta-cells), as well as genetically or otherwise modified derivatives of said cell types or progeny thereof.

Also provided are downstream derivatives of the herein taught cell types and cell populations, including without limitation: isolated nucleic acids (*e.g*., DNA, total RNA or mRNA), isolated or cloned DNA or cDNA, isolated proteins or antigens, isolated lipids, or isolated extracts (*e.g*., nuclear, mitochondrial, microsomal, *etc*.) from said cells or cell populations.

The invention also provides compositions comprising the herein taught isolated cells or cell populations, in particular isolated pancreatic progenitor cells, pancreatic endocrine progenitor cells and/or pancreatic endocrine cells (for example beta-cells) or isolated cell populations comprising any one or more of said cell types.

Apart from said cells and/or cell populations, such composition may comprise one or more other components. For example, components may be included that can maintain or enhance the viability of the cells and/or cell populations. By means of example and without limitation, such components may include salts to ensure substantially isotonic conditions, pH stabilisers such as buffer system(s) (e.g., to ensure substantially neutral pH, such as phosphate or carbonate buffer system), carrier proteins such as for example albumin, media including basal media and/or media supplements, serum or plasma, nutrients, carbohydrate sources, preservatives, stabilisers, anti-oxidants or other materials well known to those skilled in the art.

Also disclosed are methods of producing said compositions by admixing the herein taught isolated cells or cell populations with one or more additional components as above. The compositions may be for example liquid or may be semi-solid or solid (e.g., may be frozen compositions or may exist as gel or may exist on solid support or scaffold, etc.). Cryopreservatives such as *inter alia* DMSO are well known in the art.

In an embodiment, the composition as defined herein may be a pharmaceutical composition. Said pharmaceutical composition may thus comprise the herein taught isolated cells or cell populations, in particular isolated pancreatic progenitor cells, pancreatic endocrine progenitor cells and/or pancreatic endocrine cells (for example beta-cells) or isolated cell populations comprising any one or more of said cell types, as the active ingredient, and one or more pharmaceutically acceptable carrier/excipient.

Also disclosed are methods of producing said pharmaceutical compositions by admixing the herein taught isolated cells or cell populations with one or more pharmaceutically acceptable carrier/excipient.

Preferably, the pharmaceutical compositions may comprise a therapeutically effective amount of the herein taught isolated cells or cell populations. The term "therapeutically effective amount" refers to an amount which can elicit a biological or medicinal response in a tissue, system, animal or human that is being sought by a researcher, veterinarian, medical doctor or other clinician, and in particular can prevent or alleviate one or more of the local or systemic symptoms or features of a disease or condition being treated.

The term "pharmaceutically acceptable" as used herein is consistent with the art and means compatible with the other ingredients of a pharmaceutical composition and not deleterious to the recipient thereof.

As used herein, "carrier" or "excipient" includes any and all solvents, diluents, buffers (such as, e.g., neutral buffered saline or phosphate buffered saline), solubilisers, colloids, dispersion media, vehicles, fillers, chelating agents (such as, e.g., EDTA or glutathione), amino acids (such as, e.g., glycine), proteins, disintegrants, binders, lubricants, wetting agents, emulsifiers, sweeteners, colorants, flavourings, aromatisers, thickeners, agents for achieving a depot effect, coatings, antifungal agents, preservatives, stabilisers, antioxidants, tonicity controlling agents, absorption delaying agents, and the like. The use of such media and agents for pharmaceutical active substances is well known in the art. Such materials should be non-toxic and should not interfere with the activity of the cells or cell populations.

The precise nature of the carrier or excipient or other material will depend on the route of administration. For example, the composition may be in the form of a parenterally acceptable aqueous solution, which is pyrogen-free and has suitable pH, isotonicity and stability. For general principles in medicinal formulation, the reader is referred to Cell Therapy: Stem Cell Transplantation, Gene Therapy, and Cellular Immunotherapy, by G. Morstyn & W. Sheridan eds., Cambridge University Press, 1996; and Hematopoietic Stem Cell Therapy, E. D. Ball, J. Lister & P. Law, Churchill Livingstone, 2000.

Liquid pharmaceutical compositions may generally include a liquid carrier such as water or a pharmaceutically acceptable aqueous solution. For example, physiological saline solution, tissue or cell culture media, dextrose or other saccharide solution or glycols such as ethylene glycol, propylene glycol or polyethylene glycol may be included.

The composition may include one or more of a pancreatic cell protective molecules, a pancreatic cell regenerative molecule, a growth factor, pancreatic duct cells, a pancreatic exocrine cell, anti-apoptotic factor, or factor that regulates gene expression in the cells of the invention. Such substances may render the cells independent of its environment.

Such pharmaceutical compositions may contain further components ensuring the viability of the cells therein. For example, the compositions may comprise a suitable buffer system (*e.g*., phosphate or carbonate buffer system) to achieve desirable pH, more usually near neutral pH, and may comprise sufficient salt to ensure isoosmotic conditions for the cells to prevent osmotic stress. For example, suitable solution for these purposes may be phosphate-buffered saline (PBS), sodium chloride solution, Ringer's Injection or Lactated Ringer's Injection, as known in the art. Further, the composition may comprise a carrier protein, *e.g*., albumin (*e.g*., bovine or human albumin), which may increase the viability of the cells.

Further suitably pharmaceutically acceptable carriers or additives are well known to those skilled in the art and for instance may be selected from proteins such as collagen or gelatine, carbohydrates such as starch, polysaccharides, sugars (dextrose, glucose and sucrose), cellulose derivatives like sodium or calcium carboxymethylcellulose, hydroxypropyl cellulose or hydroxypropylmethyl cellulose, pregeletanized starches, pectin agar, carrageenan, clays, hydrophilic gums (acacia gum, guar gum, arabic gum and xanthan gum), alginic acid, alginates, hyaluronic acid, polyglycolic and polylactic acid, dextran, pectins, synthetic polymers such as water-soluble acrylic polymer or polyvinylpyrrolidone, proteoglycans, calcium phosphate and the like.

If desired, cell preparation can be administered on a support, scaffold, matrix or material to provide improved tissue regeneration. For example, the material can be a granular ceramic, or a biopolymer such as gelatine, collagen, or fibrinogen. Porous matrices can be synthesized according to standard techniques (*e.g*., Mikos et al., Biomaterials 14: 323, 1993; Mikos et al., Polymer 35:1068, 1994; Cook et al., J. Biomed. Mater. Res. 35:513, 1997). Such support, scaffold, matrix or material may be biodegradable or non-biodegradable.

Alternatively or in addition, the present cells or cell populations may be stably or transiently transformed with nucleic acids of interest prior to introduction to the subject. Nucleic acid sequences of interest include, but are not limited to those encoding gene products that enhance the growth, differentiation and/or functioning of pancreatic cells and particularly pancreatic endocrine cells. For example, an expression system for insulin can be introduced in a stable or transient fashion for the purpose of treating diseases or conditions benefiting from administration of insulin. Methods of cell transformation are known to those skilled in the art.

In a further aspect, the invention relates to an arrangement comprising a surgical instrument or device for administration of a composition to a subject, such as for example systemically, topically, within pancreas or within another organ or tissue (*e.g*., portal vein of the liver, spleen, pancreas, kidney capsule, peritoneum and omental pouch), and further comprising the cells or cell populations of the invention, or a pharmaceutical composition comprising said cells or cell populations, wherein the arrangement is adapted for administration of the pharmaceutical composition for example systemically, topically, within pancreas or within another organ or tissue. For example, a suitable surgical instrument may be capable of injecting a liquid composition comprising cells of the present invention, such as systemically, topically, within pancreas or within another organ or tissue.

The cells or cell populations can be administered in a manner that permits them to survive, grow, propagate and/or differentiate towards desired pancreatic cell types such as pancreatic endocrine cells. The cells or cell populations may be grafted to or may migrate to and engraft within the intended organ, such as pancreas. Engraftment of the cells or cell populations in other places, tissues or organs such as liver, spleen, pancreas, kidney capsule, peritoneum or omentum may be envisaged. The cells or cell populations may also perform the desired function (e.g., production and secretion of pancreatic hormone into the blood-stream) without engrafting or integrating within an organ or tissue, e.g., they may be administered using microencapsulation techniques, *i.e.,* provided within a discrete container, vessel or pouch to be implanted, Typically, the cells may be immobilised within a polymeric matrix and surrounded by a semi-permeable membrane (*e.g*., Murua et al. 2008. J Control Release 132: 76-83; Simon Benita, "Microencapsulation", 1st ed., Informa Healthcare 1996, ISBN 0824797035)

In an embodiment the pharmaceutical cell preparation as defined above may be administered in a form of liquid composition. In embodiments, the cells or pharmaceutical composition comprising such can be administered systemically, topically, within pancreas or at a site of pancreas dysfunction or lesion.

In another embodiment, the cells or cell populations may be transferred to and/or cultured on suitable substrate, such as porous or non-porous substrate, to provide for implants. For example, cells that have proliferated, or that are being differentiated in culture dishes, can be transferred onto three-dimensional solid supports in order to cause them to multiply and/or continue the differentiation process by incubating the solid support in a liquid nutrient medium of the invention, if necessary. Cells can be transferred onto a three-dimensional solid support, e.g. by impregnating said support with a liquid suspension containing said cells. The impregnated supports obtained in this way can be implanted in a human subject. Such impregnated supports can also be re-cultured by immersing them in a liquid culture medium, prior to being finally implanted. The three-dimensional solid support needs to be biocompatible so as to enable it to be implanted in a human. It may be biodegradable or non-biodegradable.

Cells and cell cultures may be implanted or transplanted into a patient by any technique known in the art, e.g., as discussed above and further explained *inter alia* in Robert J. Corry; Ron Shapiro, "Pancreatic Transplantation", 1st ed., Informa Healthcare 2006, ISBN 0824728793; Rainer W.G. Gruessner, David E.R. Sutherland, "Transplantation of the Pancreas", 1st ed., Springer 2004, ISBN 0387005897; James Shapiro, James A.M. Shaw, "Islet Transplantation and Beta Cell Replacement Therapy", 1st ed., Informa HealthCare 2007, ISBN 0824728629; Merani et al. 2008 (Br J Surg 95: 1449-61); etc.

Further, cells and cell cultures taught herein may be included in bio-artificial pancreas or pancreas assist devices (see, e.g., Kobayashi N. 2008. Cell Transplant 17: 11-7).

Where administration of cells or cell populations as taught herein to a patient is contemplated, it may be preferable that the cells or cell cultures are selected such as to maximise the tissue compatibility between the patient and the administered cells, thereby reducing the chance of rejection of the administered cells by patient's immune system (graft vs. host rejection). For example, advantageously the cells or cell cultures may be typically selected which have either identical HLA haplotypes (including one or preferably more HLA-A, HLA-B, HLA-C, HLA-D, HLA-DR, HLA-DP and HLA-DQ; preferably one or preferably all HLA-A, HLA-B and HLA-C) to the patient, or which have the most HLA antigen alleles common to the patient and none or the least of HLA antigens to which the patient contains pre-existing anti-HLA antibodies.

The invention further contemplates the use of the herein taught isolated cells or cell populations, in particular isolated pancreatic progenitor cells, pancreatic endocrine progenitor cells and/or pancreatic endocrine cells (for example beta-cells) or isolated cell populations comprising any one or more of said cell types, for use in therapy or for use as a medicament. In particular, the cells and cell populations are contemplated for use in the treatment of conditions or diseases associated with impairment or dysfunction of endocrine pancreas (e.g., associated with absent or suboptimal expression of one or more pancreatic endocrine hormones), conditions or diseases associated with impaired beta-cell function (e.g., associated with absent or suboptimal expression of insulin), conditions or diseases associated with impaired glucose homeostasis, prediabetes or diabetes.

Also contemplated is the use of said cells or cell populations for the manufacture of a medicament for the treatment of said conditions or diseases. Also contemplated is a method for treating a condition or disorder as above in a patient in need of such treatment, comprising administering a therapeutically effective amount of the isolated cells or cell populations as taught herein to said patient. For instance, said isolated cells may be transplanted or injected to the patient as disclosed elsewhere in this specification.

Except when noted, "subject" or "patient" are used interchangeably and refer to animals, preferably vertebrates, more preferably mammals, and specifically includes human patients and non-human mammals. Accordingly, "subject" or "patient" as used herein means any animal, mammalian or human patient or subject to which the compositions of the invention can be administered. Preferred patients are human subjects.

As used herein, the terms "treat" or "treatment" refer to both therapeutic treatment and prophylactic or preventative measures, wherein the object is to prevent or slow down (lessen) an undesired physiological change or disorder, such as the development of a disorder of glucose homeostasis, e.g., diabetes type 2. Beneficial or desired clinical results include, but are not limited to, alleviation of symptoms, diminishment of extent of disease, stabilised (i.e., not worsening) state of disease, delay or slowing of disease progression and occurrence of complications, amelioration or palliation of the disease state. "Treatment" can also mean prolonging survival as compared to expected survival if not receiving treatment. Without limitation, a therapeutic endpoint may be adequate glycaemic control (e.g., HbA1c<7%), increased insulin levels, etc.

As used herein, a phrase such as "a subject in need of treatment" includes subjects, such as mammalian or human subjects, that would benefit from treatment of a given condition, preferably a condition or disease as above. Such subjects will typically include, without limitation, those that have been diagnosed with the condition, those prone to have or develop the said condition and/or those in whom the condition is to be prevented.

The cells or cell populations of the invention may be used alone or in combination with any of the known therapies for disorders of glucose homeostasis, e.g., selected from the group comprising therapeutics for combating disorders involving hyperglycaemia, such as without limitation, insulin, biguanides (e.g., metformin), thiazolidinediones (TZD), sulfonylurea and derivatives thereof, or incretin (GLP1 agonists). The cells or cell populations of the invention can thus be administered alone or in combination with one or more active compounds. The administration may be simultaneous or sequential in any order.

Conditions, diseases or disorders the treatment of which using the cells or cell populations as taught herein is intended may include *inter alia* disorders of glucose homeostasis.

"Disorders of glucose homeostasis" encompass all disorders in which disturbance of physiologically normal homeostasis of circulating glucose belongs to, *i.e.,* is comprised amongst, symptoms of the said disorders, and/or belongs to aetiological factors of the said disorders. The diagnosis of a disorder of glucose homeostasis in a subject, *i.e.,* the differentiation between physiologically normal vs. abnormal glucose homeostasis in the said subject, may be done by a number of methods well-known in clinical practice, including but not limited to measurement of fasting glucose level; and oral or intravenous glucose tolerance test.

A typical fasting blood glucose test measures the concentration of glucose in blood, serum or plasma of a subject after a fasting period of usually at least 10-12 hours. The normal range of whole blood glucose concentrations (normoglycaemia), indicative of physiologically normal glucose homeostasis, in this test is between 60 mg/dL (3.0 mmol/L) and 110 mg/dL (5.6 mmol/L).

A typical oral glucose tolerance test (OGTT) is carried out as follows: after an overnight fasting period (e.g., 10 to 12 hours), a subject drinks a solution containing a known amount of glucose; blood is drawn before the subject drinks the glucose solution, and blood is drawn again every 30 to 60 minutes after the glucose solution is consumed for up to 3 hours. The normal ranges of whole blood glucose concentrations in a 75-gram oral glucose tolerance test (normoglycaemia), indicative of physiologically normal glucose homeostasis, are: between 60 mg/dL (3.0 mmol/L) and 110 mg/dL (5.6 mmol/L) after fasting; less than 200 mg/dL (10.1 mmol/L) at 1 hour after consumption of the glucose solution; less than 140 mg/dL (7.1 mmol/L) at 2 hours after consumption of the glucose solution.

Accordingly, a disorder of glucose homeostasis is diagnosed when glucose concentrations in at least one and preferably both above tests, or analogous tests commonly employed in the art, fall outside of the normoglycaemic ranges, e.g., as indicated above, at least on one occasion and preferably on two or more occasions.

In a preferred embodiment, said condition or disease may be associated with hyperglycaemia. Typically, a diagnosis of hyperglycaemia as a disorder of glucose homeostasis or as a symptom of said disorder is made if the measured glucose levels are above their normal ranges.

By means of example and not limitation, in the above fasting blood glucose test, a diagnosis of impaired fasting glucose (IFG) is diagnosed when the whole blood glucose concentration of a subject is above 110 mg/dL (5.6 mmol/L) but less than 126 mg/dL (6.4 mmol/L). Diabetes mellitus may be diagnosed when the whole blood glucose concentration of a subject is above 126 mg/dL (6.4 mmol/L).

By means of example and not limitation, in the above OGTT, impaired glucose tolerance (IGT) is diagnosed when the whole blood glucose concentration of a subject at 2 hours after consumption of the glucose solution is higher than 140 mg/dL (7.1 mmol/L) but less than 200 mg/dL (10.1 mmol/L). Diabetes mellitus may be diagnosed when the whole blood glucose concentration of a subject at 2 hours after consumption of the glucose solution is higher than 200 mg/dL (10.1 mmol/L).

When at least one of IFG and IGT, and preferably both IFG and IGT, are diagnosed, the condition may be referred in the art as "prediabetes". In a preferred embodiment, said condition or disease may be prediabetes.

In a preferred embodiment, said condition or disease may be a disorders of glucose homeostasis encompassing hyperglycaemia; IGT; IFG; prediabetes; diabetes, including diabetes types 1 and 2; metabolic syndrome and the like.

The terms "metabolic syndrome", "insulin resistance syndrome" (IRS), or "syndrome X" may be used interchangeably herein and refer to a cluster of abnormalities which tend to co-occur in a subject and which represent major risk factors for the development of coronary artery disease (CAD), such as premature atherosclerotic vascular disease. These abnormalities in particular involve insulin resistance (impaired blood glucose), truncal obesity, high serum low density lipoprotein (LDL) cholesterol levels, low serum high density lipoprotein (HDL) cholesterol levels, high serum triglyceride levels, and high blood pressure (hypertension). According to the National Cholesterol Education Program of NIH, diagnosis of metabolic syndrome is made in the presence of any three of the following abnormalities: truncal obesity, defined as waist circumference of more than 102 cm for men and more than 89 cm for women; high serum levels of triglycerides, i.e., 150 mg/dL or higher; low serum levels of HDL cholesterol, i.e., below 40 mg/dL for men and below 50 mg/dL for women; high blood pressure, i.e., 130/85 mm Hg or higher; impaired fasting glucose, i.e., 110 mg/dL or higher.

Primary diabetes mellitus (DM) is classified as type 1 diabetes (also called juvenile onset DM or insulin dependent diabetes mellitus, IDDM) and type 2 diabetes mellitus (also called non-insulin dependent diabetes mellitus, NIDDM). Type 1 diabetes is a hormone deficient state, in which the pancreatic beta cells appear to have been destroyed by the body's own immune defense mechanisms. The destruction of beta cells in type 1 diabetes leads to the inability to produce insulin, and thereby chronic insulin deficiency. Patients with type 1 diabetes have little or no endogenous insulin secretory capacity and develop extreme hyperglycemia. Type 1 diabetes was fatal until the introduction of insulin replacement therapy - first using insulins from animal sources, and more recently, using human insulin made by recombinant DNA technology.

Type 2 diabetes mellitus (referred to herein as "type 2 diabetes") is typically a chronic, lifelong (i.e., progressing over several decades) disease characterized by insulin resistance. In clinical terms, insulin resistance is present when normal or elevated blood glucose levels persist in the face of normal or elevated levels of insulin. Symptoms may include excessive thirst, frequent urination, hunger, and fatigue. Typically, type 2 diabetes may be diagnosed when the fasting blood glucose concentration of a subject is above 126 mg/dL (6.4 mmol/L) on two occasions. Type 2 diabetes may also be diagnosed using OGTT when the blood glucose concentration of a subject at 2 hours after consumption of the glucose solution is higher than 200 mg/dL (10.1 mmol/L). Hyperglycaemia associated with type 2 diabetes can sometimes be reversed or ameliorated by diet changes or weight loss which may at least partially restore the sensitivity of the peripheral tissues to insulin. Treatment of type 2 diabetes frequently does not require the use of insulin. Therapy in type 2 diabetes usually involves dietary therapy and lifestyle modifications, typically for 6-12 weeks in the first instance. Features of a diabetic diet include an adequate but not excessive total calorie intake, with regular meals, restriction of the content of saturated fat, a concomitant increase in the polyunsaturated fatty acid content, and an increased intake of dietary fiber. Lifestyle modifications include the maintenance of regular exercise, as an aid both to weight control and also to reduce the degree of insulin resistance. If after an adequate trial of diet and lifestyle modifications, fasting hyperglycemia persists, then a diagnosis of "primary diet failure" may be made, and either a trial of oral hypoglycaemic therapy or direct institution of insulin therapy may be required to produce blood glucose control and, thereby, to minimize the complications of the disease. Progression of type 2 diabetes is associated with increasing hyperglycemia coupled with a relative decrease in the rate of glucose-induced insulin secretion. Therefore, for example, in late-stage type 2 diabetes there may be an insulin deficiency.

In a preferred embodiment, said condition or disease may be a diabetes, including diabetes types 1 and 2.

In further aspects, the herein taught isolated cells or cell populations, in particular isolated pancreatic progenitor cells, pancreatic endocrine progenitor cells and/or pancreatic endocrine cells (for example beta-cells) or isolated cell populations comprising any one or more of said cell types, may represent *in vitro* models for pathological conditions and diseases, particularly human diseases, such as mentioned above.

For example, said cells and cell populations may be derived from subjects having a disease of interest, or the cells or populations may be derived from healthy subjects and further manipulated to display a pathological phenotype of interest. For example, such manipulation may include contacting said cells or populations externally with an agent, *e.g*., a chemical or biological agent, known or suspected of causing a pathological phenotype of interest. Exemplary agents may include, without limitation, toxins, metabolites, drugs, antisera, viral agents etc. In another example, such manipulation may include transiently or stably transforming the cells (*e.*g., by transfection or transduction as known in the art) with a recombinant construct encoding an RNA or protein agent known or suspected of causing a pathological phenotype of interest, or an agent (*e.g*., an RNAi agent or a dominant negative variant) that can suppress the expression of an endogenous gene known or suspected to contribute to a disease of interest.

In another aspect, the invention provides use of the herein taught cells or cell populations, optionally and preferably wherein said cells or populations represent models for conditions or diseases taught herein, particularly human diseases, in any variety of screening assays, particularly *in vitro* screening assays, such as, e.g., in assays of biological effects of candidate pharmacological substances and compositions; assays of cellular toxicity, genotoxicity or carcinogenicity of chemical or biological agents; assays allowing the study of normal pancreatic endocrine development, function and of the aetiology of pancreatic endocrine diseases, and the like.

Cell-based *in vitro* screening assays can be carried out as generally known in the art. For example, cells grown in a suitable assay format (*e.g*., in multi-well plates or on coverslips, *etc*.) are contacted with a candidate agent (*e.g*., a potential pharmacological agent) and the effect of said agent on one or more relevant readout parameters is determined and compared to a control. Relevant readout parameters may greatly vary depending on the type of assay and may include, without limitation, survival, occurrence of apoptosis or necrosis, altered morphology, altered hormone or enzyme secretion, altered responsiveness to external signals or metabolites, gene expression, *etc*. Hence, in an embodiment the invention provides a screening assay to identify pharmacological agents for the treatment of a condition or disease as discussed herein, comprising contacting the cells or cell populations taught herein with a candidate pharmacological agent, and determining alleviation of said disease phenotype when said agent is administered. The invention also relates to so-identified pharmacological agents.

Accordingly, the invention also provides the use of cells or cell populations of the invention for screening assays preferably *in vitro* screening assays, particularly pharmaceutical, genetic or toxicological screening assays. In an embodiment, said cells or cell populations may represent (*e*.*g*., have been so manipulated, see above) a model for a condition or disease as intended herein, preferably a human disease.

For example, the present pancreatic progenitors or cell populations comprising such, or preferably pancreatic endocrine progenitors or cell populations comprising such, may be used to screen for agents or factors (*e*.*g*., small molecules, chemokines, growth factors, etc.) which induce differentiation towards pancreatic endocrine cells, more preferably towards beta-cells, or to screen for phenotypic changes (*e*.*g*., markers, gene expression, cell morphology, etc.) that typify such differentiation.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

The above aspects and embodiments are further supported by the following non-limiting examples.

### EXAMPLES

The following examples demonstrate *inter alia* the following facts. Firstly, a sequential cocktail of factors reported by D'Amour *et al.* 2006 (*supra*) as inducing pancreatic cells from human embryonic stem cells generated mainly cells with hepatic phenotype and functioning as mature hepatocytes. Such hepatocyte-like cells were reproducibly obtained from four human embryonic stem cell lines previously derived and maintained by the Applicant, at the expense of pancreatic differentiation (see Examples 2-4). Next, the examples establish that the hepatic differentiation obtained by the protocol of D'Amour *et a*/*.* 2006 (*supra*) is partly associated with early treatment of the cells with FGF10, and is negatively regulated by BMP and FGF pathways inhibition (Example 5). The Applicant next made use of the above information to devise a method in which hepatogenesis is inhibited and pancreas initiation is induced (Example 6). Furthermore, the Applicant also demonstrates methods to further amplify and differentiate the so-obtained early pancreas progenitors towards endocrine progenitors by exposure to FGF ligands and Notch pathway inhibitors (Example 7).

### Example 1: Experimental procedures

### Human ES cell culture and differentiation

Human hES cell lines (VUB01, VUB02, VUB07, VUB14) were generated and characterized at our institute as described in Mateizel et al. 2006 (Hum. Reprod. 21: 503-511). VUB01, VUB02, and VUB07 are deposited at www.hESCreg.eu (European Human Embryonic Stem Cell Registry). Undifferentiated cells were maintained on feeder layers made of Mitomycin-inactivated or irradiated mouse embryonic fibroblasts (Millipore and RnD Systems respectively). The culture medium consisted of knockout DMEM (Invitrogen) supplemented with 20% knockout serum replacement (Invitrogen), 0.55mM 2-mercaptoethanol (Sigma), 1mM non-essential amino acids (Sigma), Glutamax (Invitrogen), Penicillin-Streptomycin (Sigma), and 4 ng/ml recombinant FGF2 (RnD Systems). Cultures were manually passaged every 5-6 days at a 1:3-1:6 split ratio and quality control carried out at intervals by alkaline phosphatase assay and karyotype analysis as described in Mateizel et al. 2006 (*supra*).

Cells were split in 4-well plates and differentiation performed in five stages as previously published (D'Amour et al. 2006 (*supra*); Table 2). Briefly, for DE induction 80% confluent cultures were washed with PBS and treated with 100 ng/ml Activin-A (RnD Systems) and 25 ng/ml Wnt3a (RnD Systems) for 24h in RPMI (Invitrogen) supplemented with antibiotics and Glutamax; then they were further treated with 100 ng/ml Activin-A in RPMI containing 0.2% FBS (PAA Laboratories) for 48h. PGT stage cells were treated with 50 ng/ml FGF10 (RnD Systems), 250 nM KAAD-Cyclopamine (Calbiochem) and 2% FBS in RPMI medium for 3 or 4 days. In the PFG stage (3-4 days), the medium was changed to DMEM supplemented with Glutamax, antibiotics, 1% B27 (Invitrogen), 2 µM retinoic acid (RA, Sigma), FGF10 and KAAD-Cyclopamine. The cells were further exposed to the gamma-secretase inhibitor Compound-E (50 µM; Calbiochem) and Exendin-4 (50 ng/ml; Sigma) for 4 days (PEEP stage), and finally to CMRL medium (Invitrogen) supplemented with Glutamax, antibiotics, 1% B27, IGF1 (50 ng/ml; Sigma), HGF (50 ng/ml; RnD Systems) and Exendin-4 for at least 4 days (HEEC stage).

As data from our cell lines did not indicate any success in pancreas differentiation, we introduced several modifications to the original protocol, focusing on PGT, PFG and PEEP stages. The initial modifications consisted of withdrawing FGF10 from the PFG stage, or from both PGT and PFG stages while maintaining all the other components. In order to inhibit FGF signaling, cells were treated with 2.5 µM SU5402 (Calbiochem) in the PGT and PFG stages. This was combined or not with BMP antagonist Noggin (100 ng/ml; RnD Systems) during the same period. In another set of experiments, the effects of different supplements were tested by single or combined supplementation of chemicals and growth factors used in PGT and PFG stages (FGF10, KAAD-cyclopamine and RA), and by BMP antagonism in the PGT stage (Fig. 4a). Finally, exposure to RA was performed much earlier (PGT) and FGF10 treatment much later (PEEP), giving rise to three modified protocols referred to as P1, P2 and P3 (Table 1 and Table 2).

### Gene expression analysis by PCR

Total RNA was extracted from two wells per sample at the end of each stage using a column RNA binding kit (Sigma) as recommended by the manufacturer. After DNase I (Sigma) treatment, reverse transcription was carried out with random hexamers (3:4) and oligo-dT (1:4) from 500-750 ng RNA using the Verso cDNA kit (Thermo-Scientific). For quantitative PCR, 10 µl cDNA (35-50 ng RNA-equivalent) was used together with 250 nM forward and reverse primers in 1x final concentration of Absolute QPCR SYBR Green I master mix (Thermo-Scientific). The primers used were from D'Amour et al. 2006 *(supra).* For the analysis of liver markers, additional ones were designed to span introns whenever possible. Relative quantification of gene expression was performed following the ddCt method with TBP taken as endogenous control. Data (mean + s.e.m of at least 3 independent experiments) were plotted as a ratio to undifferentiated hES cell expression level set at 1 (Figures 1A, 1E, 1I, 2C, 3C, 4C). Experiments performed according to protocols P2 and P3 were also analyzed similarly, but data are presented as fold-change versus levels recorded in the original protocol (Figure 5E).

### Immunofluorescence and immunocytochemistry

At each stage, cultures were washed with PBS, fixed for 10-15 min at room temperature in 4% formaldehyde and rinsed several times. Occasionally, cells were dissociated with TrypZean (Sigma) after PBS wash and cytospins prepared on glass slide. These were fixed in 4% formaldehyde for 5 min and rinsed several times with PBS. Cells were treated for 20 min in 99% methanol at -20°C for permeabilisation, and then blocked for 30 min in 2% donkey serum. Primary antibodies were diluted in PBS and incubated for 24 h at 4°C. The following primary antibodies and dilutions were used: mouse anti-Sox17, 1:100 (RnD Systems, MAB1924); mouse anti-CxCR4, 1:100 (RnD Systems, MAB171); goat anti-Foxa2, 1:100 (Santa Cruz Biotechnology, SC-9187); goat anti-Hnf1B, 1:100 (Santa Cruz Biotechnology, SC-7411); rabbit anti-Hnf6, 1:100 (Santa Cruz Biotechnology, SC-6559); rabbit anti-Sox9, 1:500 (Chemicon, AB5535); goat anti-Pdx1, 1:100 (RnD Systems, AF2419); mouse anti-Glucagon, 1:100 (Sigma, G2654); guinea-pig anti-Insulin, 1:3000 (Prof. CV Schravendijk, VUB, Belgium); rabbit anti-Amylase, 1:500 (Sigma, A8273); rabbit anti-AFP, 1:400 (Dako, A008); mouse anti-Albumin, 1:200 (Sigma, A6684); and rabbit anti-CEBPB, 1:100 100 (Santa Cruz Biotechnology, SC-150). Cy2-, FITC-, and TRITC-conjugated secondary donkey antibodies (Jackson ImmunoResearch Laboratories) were also diluted in PBS and incubated for 1 h at room temperature, followed by DNA staining with DAPI for 15 min. All secondary antibodies were used at 1:100 dilutions, except for anti-guinea-pig (1/200) and anti-rabbit (1:400). Immunocytochemistry was performed for the detection of Hnf1B using a donkey horse radish peroxidase (HRP)-conjugated secondary antibody and detected by the ABC complex (Dako) as previously described in Mfopou et al. 2007 (Stem Cells 25: 1156-1165).

### Periodic acid Shiff (PAS) staining

At the end of HEEC stage, differentiated cells were washed with PBS and fixed for 10 min in 10% paraformaldehyde at room temperature. Cells were rinsed and incubated with periodic acid (Sigma) for 5 min, then in Shiff reagent (Sigma) for 10-15 min to develop the pink color. Stained wells were examined immediately after staining by bright field microscopy.

### Microscopy

Bright field and fluorescent images were acquired from 4-well plates and cytospin slides using an inverted microscope (Nikon TE2000E) and the NIS Elements AR2.30 imaging software. Estimation of positive area ratios was performed by manually setting a treshold for each channel and allowing the software to compute the total positive area for DAPI, TRITC and FITC. The values recorded for FITC and TRITC were divided by that of DAPI to get the ratio.

### Western blot

Protein extracts were collected from undifferentiated or differentiated cells using Laemmli buffer and 25 µg loaded on 10% polyacrylamide gels (Bio-Rad) for electrophoresis. Separated proteins were electro-blotted on nitrocellulose membranes and further processed on SNAPid system (Millipore). Briefly, membranes were blocked for 2 min with 5% rat serum and washed, then exposed to primary antibodies (same as for immunofluorescence) for 10 min at the following dilutions: goat anti-Pdx1, 1:500; goat anti-Foxa2, 1:500; mouse anti-Albumin, 1:1000; rabbit anti-AFP, 1:2000; and goat anti-Actin, 1:500 (Santa Cruz Biotechnology, SC-1616). Membranes were washed and further exposed for 10 min to HRP-conjugated donkey secondary antibodies (DakoCytomation) as follows: anti-goat: 1:2500; anti-mouse, 1:1000; anti-rabbit, 1:1000. After several rounds of rinsing, detection was performed by chemiluminescence on Kodak films and densitometric analysis of protein bands was done using Image J software (http://rsbweb.nih.gov/ij/).

### Albumin ELISA assay

Supernatants were harvested from cultures at different stages and stored at -20°C until analysis. Samples were cleared by centrifugation (3 min, 1500 rpm) and indirect ELISA performed in triplicates using a kit specific for human Albumin (Bethyl Laboratories, E80-129) that detects in the range of 6.25-400 ng/ml. Some samples collected as from PEEP were diluted 10 times to get values in the detection range of the kit. Briefly, ELISA-grade 96-well plates were coated for 1 h with goat anti-human Albumin antibody (100 µl; 10 µg/ml) diluted in 0.05 M carbonate-bicarbonate buffer (pH 9.6). The coated plates were blocked for 1 h with 200 µl blocking solution (50 mM Tris, 0.14 M NaCl, 1% BSA, pH 8.0) and incubated for 1 h with 100 µl standards (0-300 ng/ml) or samples. HRP-conjugated anti-human Albumin (1:50000) was added for and additional hour, followed by the TMB substrate (Sigma) for 5 to 15 min. Reaction was stopped by addition of 50 µl H2SO4 (1 M) and OD detection performed on Model 680 plate reader (Bio-Rad) at 450 nm. Data were analyzed with Prism software (Graphpad) using the 4-parameter logistic curve-fit to create the standard curve.

### Urea assay

Cells at the end of HEEC stage were cultured overnight in medium supplemented with 10 mM ammonium chloride (Sigma) and supernatant collected for urea assay (Gentaur). This assay was performed in 96-well plates with triplicates of each sample and standards as recommended by the manufacturer. OD were read at 490 nM (Model 680 plate reader, Bio-Rad) after 30 min incubation with the substrate and data analyzed using a linear regression model of the standard curve.

### Example 2: Differentiation of definitive endoderm

Human embryonic stem cells were maintained on mitomycin-inactivated mouse embryonic fibroblasts as feeder layers and manually passaged every 5 to 6 days. The undifferentiated embryonic stem cells grew as compact colonies with sharp borders and stained positive in the alkaline phosphatase assay. At 80% confluence, undifferentiated embryonic stem cells were washed with a buffered saline and induced to differentiate towards definitive endoderm germ layer by treatment with Wnt3a (Wingless-type MMTV integration site family, member 3A) (25 ng/ml) and Activin A (100 ng/ml) for 24h followed by Activin A and 0.2% FBS for 2 days (D'Amour *et al.* 2006, *supra*). Occasionally upon examination of cultures, the said combinations of growth factors were given for 2 days or for 3 days respectively. The definitive endoderm germ layer is usually characterized by its epithelial morphology in culture, but more importantly by the transient expression of Bry on day 1 (mesendoderm marker), which is followed by expression of Sox17, Gsc, Foxa2 and CxCR4 (Kubo et al. 2004, Development 131: 1651-1662; Tada et al. 2005, Development 132: 4363-4374; Yasunaga et al. 2005, Nature Biotechnology 23: 1542-1550; D'Amour *et a*/*.* 2006, *supra*; Jiang et al. 2007, Stem Cells 25: 1940-1953; Philips et al. 2007, Stem Cells and Development 16: 561-578). As extensively described in the literature, transcriptional activation of all these genes in the expected sequence demonstrates that definitive endoderm was induced following exposure of our undifferentiated stem cells lines to the above-mentioned growth factors for at least 3 days (Figure 1A). Furthermore, co-localisation of Sox17 with Foxa2 (60-80%; Figure 1B) and membrane expression of CxCR4 (Figure 1C) that were detected by immunoreactivity, as well as the absence of Sox7 (SRY (sex determining region Y)-box 7) expression further confirmed the definitive endoderm phenotype of the obtained cells.

### Example 3: Characterisation of differentiated cells in subsequent stages

In the subsequent stages of differentiation, cells were treated with combinations of growth factors and chemicals as reported in D'Amour et al. 2006 (*supra*) in order to produce *in vitro* equivalents of primitive gut tube (PGT), posterior foregut (PFG), pancreas endoderm - endocrine progenitors (PEEP) and hormone-expressing endocrine cells (HEEC). Because our data do not reproduce those of the original protocol, the *in vitro* equivalents mentioned here (PGT, PFG, PEEP and HEEC) mainly refer to time intervals and not to the phenotypical or molecular characteristics of the cells in cultures.

Several markers were analysed by RT-PCR to confirm the transition from one stage to the next, including Foxa2, CxCR4, Gsc, Sox17 for definitive endoderm; Foxa2, Hnf1b (hepatocyte nuclear factor 1 homeobox B) for the PGT stage; Foxa2, Hnf1 b, Hnf6, Hlxb9 for the PFG stage; Foxa2, Pdx1, Hlxb9, Nkx6.1, Ngn3, Pax4 (paired box 4) for the PEEP stage; and insulin, glucagon, amylase for the HPC stage (D'Amour *et al.* 2006, *supra*). As depicted in Figure 1D, expression of the primitive gut tube marker Hnf1β was induced at expected time point and persisted in the subsequent stage. Similarly, Hnf6 transcripts further increased at the posterior foregut stage together with Hnf1β, while Foxa2 was maintained and Sox17 decreased (Figure 1E). At the posterior foregut stage, immunoreactivity for Foxa2 was detectable in several cell clusters, whereas Pdx1-positive cells, occurring also in small clusters, were not frequently observed. These clusters represented no more than 5-10% of cells in culture (Figure 1F, 1G). The profile of all these markers is concordant with the occurrence of stage transitions, but at most up to the PFG stage. In other words, our cell lines could give rise to in vitro equivalents of PGT and PFG cells, but not enough PEEP cells could be differentiated from them. Indeed, pancreatic and endocrine progenitor markers such as Pdx1, Sox9, Nkx6.1, Ngn3 and Pax4 were detected only at low levels by PCR.

The failure to detect a high proportion of pancreatic cells from our cultures led to the examination of alternative phenotypes that can be derived from definitive endoderm, with a major focus on hepatocytes which share a common progenitor with pancreatic cells (Deutsch et al. 2001, Development 128: 871-881). Exposure of human embryonic stem cells-derived definitive endoderm to ligands of FGF receptor (FGF10, 50 ng/ml) in combination with the hedgehog pathway antagonist KAAD-cyclopamine (250 nM) for three days induced a high proportion (> 40%) of cells expressing alpha-fetoprotein (AFP) with or without low levels of albumin (ALB) (Figure 1H). After three other days of exposure to FGF10 combined with KAAD-Cyclopamine and retinoic acid, the proportions as well as the signal intensities for both ALB and AFP further increased, so that the majority of cells were double positive for these markers (Figure 1H). Transcript analysis confirmed the sharp increase in AFP and ALB expression when compared to levels in undifferentiated human embryonic stem cells (Figure 1I). The expression pattern of these genes recalls early liver progenitor cells, namely the hepatoblasts. In sharp contrast to the abundant expression of AFP and albumin, pancreas markers and notably Pdx1 were barely detected both at transcripts and at protein levels, and represented only minute amounts of levels found in human pancreas.

Although these findings deviated from our initial expectations, they urged the further analysis of liver differentiation in the system. At the PEEP stage, the AFP-ALB double positive hepatoblasts represented about 70% of cells in culture. The ALB+ cells observed at the PEEP stage persisted till the HEEC stage; they also co-express Foxa2 at variable levels (Figure 2A). In the last stage (HEEC), these cells have lost AFP expression and significantly reduced their expression of Foxa2 while maintaining ALB at high levels (Figure 2A, 2B). Further analysis indicated induction of other liver genes such as CEBPβ (CCAAT/enhancer binding protein beta), glucose-6-phosphatase catalytic unit, alpha-1-antitrypsin and transthyretrin (Figure 2C, 2D). Taken together, these data strongly suggest a progressive transition from immature AFP+ALB+ hepatoblasts towards mature AFP-ALB+ hepatocytes, and this at the expense of cells with a pancreas phenotype.

### Example 4: Functional assessment of hepatic differentiation

*In vivo,* mature liver cells undertake several physiological functions including but not limited to the synthesis and secretion of albumin in serum, the storage of excess carbohydrate in the form of glycogen, and the excretion of numerous substances whose accumulation might induce toxic syndromes. We sought to determine the maturity level of hepatocyte-like cells differentiated from human embryonic stem cells following the above-mentioned method. We examined the detoxification function of these hepatocyte-like cells by culturing them overnight in the presence of 10 mM ammonium chloride. In the supernatant collected from differentiated cells, urea was detected at much higher levels (1.3 mg/dl versus < 0.1 mg/dl) compared to that in non-exposed medium (Figure 2E). Using an ELISA assay that specifically detects human albumin, we demonstrated a progressive increase in the amount of albumin released in the culture medium by differentiated cells. At the PEEP stage, albumin release (7 ng/ml in 48h-exposed medium) was significantly higher compared to the PGT stage when hepatoblasts are established. At the HEEC stage, the release was further increased 5 folds (35 ng/ml in 48h-exposed medium), which was concordant with additional data suggesting hepatocyte maturation (Figure 2F). In addition, using a periodic acid Schiff assay, we also demonstrated that many differentiated cells, some of which were binucleated, stored glycogen in their cytoplasm (Figure 2G). All these data confirm that the majority of cells obtained in these cultures were hepatocyte-like cells and functioned accordingly.

### Example 5: Effects of FGF and BMP pathways on hepatic differentiation

During development, BMP and FGF signals from the septum transversum mesenchyme and the cardiac mesoderm regulate hepatic competence and specification of the gut endoderm (Rossi et al. 2001, Genes Dev. 15: 1998-2009; Zaret 2002, Nat. Rev. Genet. 3: 499-512; Berg et al. 2007, Hepatology 46: 1187-1197. We hypothesised that FGF10 supplementation to nascent definitive endoderm in vitro might also play such a function and result in hepatic differentiation as we observed. We therefore initiated several modifications, starting with the omission of FGF10 supplementation in the PGT, the PFG or both stages. Our data indicated that FGF10 removal from the system only partially reduced the extent of hepatic differentiation. More interestingly, addition of FGF10 during the PGT stage but not in the PFG stage led to an early switch from ALB+AFP+ to AFP-ALB+ cells, occurring already in the PEEP stage (Figure 3A). Inhibition of this pathway by addition of SU5402 reduced hepatic differentiation by at least 50%, and combined inhibition of the BMP pathway by addition of noggin almost completely abrogated it (Figure 3B, 3C).

### Example 6: Inhibition of hepatogenesis combined with pancreas induction

We next used the information gathered from previous examples to devise a method in which hepatogenesis is blocked (no FGF10 addition, inhibition of BMP pathway with noggin) while pancreas phenotype is concomitantly induced by supplementation of cyclopamine and retinoic acid to nascent definitive endoderm cells. This stage is referred to herein as HBPI (hepatic blockade and pancreas induction) and corresponds to the total length of PGT and PFG in the protocol of D'Amour *et al.* 2006 *(supra).* This treatment (protocol P3, see Table 1 and 2) was continued for 7 to 8 days and resulted in nearly 90% of cells expressing Foxa2 at high levels and in large monolayers or aggregates (Figure 4A). Many of these cells were Sox9 positive, but the degree of Sox9 expression varied between cells. Pdx1 expression was also detected in much larger clusters (about 30% cells) and at higher intensity when compared to the D'Amour protocol (Figure 4B). Concomitantly, albumin expression was lost in these conditions (Figure 4A). The beneficial effects of the new methods, both in terms of loss of albumin and gain of Foxa2 and Pdx1 were confirmed by quantitative PCR analysis (Figure 4C).

An alternative protocol (P2, see Table 2) was also performed, in which retinoic acid was not added during the first two days of HBPI. This treatment did not significantly affect the proportion of Pdx1-positive cells or Pdx1 transcripts expression detected at the end of HBPI, but limited the extent of Foxa2 increase (Figure 4C).

In another alternative protocol (P1, see Table 2), cells received only noggin and cyclopamine in the first 3-4 days after definitive endoderm induction, then cyclopamine and retinoid acid in the last 4 days of HBPI. This treatment induced Pdx1+ cells at the end of HBPI stage, but their proportion was limited to 30-50% of that recorded upon continuous treatment with all three factors (protocol P3).

The above described differentiation protocols are summarised in the following Tables 1 and 2.

**Table 1: Summary of the procedures and methods for efficiently generating pancreas progenitors from human embryonic stem cells. The table indicates the 3 stages of differentiation followed in this protocol, including the generation of definitive endoderm (DE), the induction of pancreas progenitor with concomitant inhibition of the liver program (HBPI), and the amplification of pancreas progenitor and induction of endocrine progenitors (PEP). The markers used to examine the cells at each stage are listed.**

| **Stages** | **Culture condition** | **Observations - Markers used** |
|---|---|---|
| Expansion of human embryonic stem cells, up to 80% confluence | Mouse embryonic fibroblasts, bFGF (4 ng/ml) | Compact colony morphology, Alkaline phosphatase assay |
| **Stage 1: DE** Induction of definitive endoderm | Activin A (100 ng/ml) + Wnt3a (25 ng/ml) for 1 day Activin A (100 ng/ml) + 0.2% FBS for 2-3 days | Cell death ++ Epithelial morphology Bry, Sox17, Foxa2, Gsc, CxCR4 |
| **Stage 2: HBPI** Hepatic blockade and pancreas induction | Noggin (100 ng/ml) + KAAD-cyclopamine (250 nM) + retinoic acid (2 µM) | AFP, Albumin, Foxa2, Sox9, Pdx1, Hlxb9, Hnf1 b |
| **Stage 3: PEP** Pancreatic and endocrine progenitors | FGF10 (50ng/ml) + compound E (1 µM) + exendin-4 (50 ng/ml) | AFP, Albumin, Foxa2, Sox9, Pdx1, Hlxb9, Hnf6, Ngn3, Nkx6.1, Ptf1a |

**Table 2: Comparison of the present protocols with the original protocol from D'Amour et a/. 2006 (supra). The present protocols make use of a hepatic blockade and pancreas induction stage which encompasses the PGT and PFG stages of the D'Amour et al. protocol and is mainly characterised by no FGF10 addition, inhibition of BMP pathway and early exposure to retinoid signalling. Three protocols are shown (P1, P2, P3) and are differentiated by the initiation and length of noggin, retinoid acid and cyclopamine treatments. Abbreviations: ActA, Activin A; FBS, foetal bovine serum; NG, noggin; CY, KAAD-cyclopamine; RA, retinoic acid; CpdE, compound E; Ex4, exendin-4.**

| **D'Amour et al. 2006** | **Present methods** | **P1** | **P2** | **P3** |
|---|---|---|---|---|
| **Stage 1: DE** Definitive endoderm | **Stage 1: DE** Definitive endoderm | ActA + Wnt3a; ActA + FBS | | |
| **Stage 2: PGT** Primitive gut tube **Stage 3: PFG** Posterior foregut | **Stage 2: HBPI** Hepatic blockade and pancreas induction | **N**G + RA | NG + CY / RA | NG + CY + RA |
| | | CY + RA | NG + CY + RA | NG + CY + RA |
| **Stage 4: PEEP** Pancreatic endoderm and endocrine progenitors | **Stage 3: PEP** Pancreatic / endocrine progenitors | FGF10 + CpdE + Ex4 | | |
| **Stage 5: HEEC** Hormone expressing endocrine cells | - | - | - | - |

### Example 7: Amplification of Pdx1+ cells and induction of endocrine program

The early pancreas progenitors obtained using the above protocols P2 and P3 were amplified and further differentiated towards endocrine progenitors by exposure to FGF10 and to a Notch pathway inhibitor acting via gamma secretase blockade (Compound E). Exposure to FGF ligand is envisaged to induce proliferation of Pdx1+ progenitors, and inhibition of Notch pathway is envisaged to induce endocrine differentiation. The glucagon-like peptide 1 agonist exendin-4 was also supplemented during this step, which is referred to as pancreas and endocrine progenitor (PEP) stage. Exposure to these factors for 4 days maintained the high expression of Foxa2 in a large proportion of cells and secured the loss of albumin and AFP expression as observed in the HBPI stage (Figure 5A). This treatment also resulted in a major increase in Pdx1+ cells representing up to 80% of cells (Figure 5B). The Pdx1+ cells co-expressed Sox9 and Hnf6 at various levels (Figure 5C, 5D), suggesting that they are of pancreatic phenotype and did not represent Pdx1+ cells observed in the developing stomach or duodenum. Quantitative PCR analysis indicated an increased expression of major pancreas markers in P2 and P3 as compared to the D'Amour *et al.* protocol, with Hnf6 and Ngn3 expression increased up to 100 and 400 fold respectively (Figure 5E). The shift from AFP- and ALB-expressing cells to Pdx1+ pancreas progenitor cells observed with the new methods was further evidenced by protein extracts analysis using corresponding antibodies (Figure 5F).

### Example 8: Application of the above protocol to additional cell lines

When tested on 3 additional cell lines VUB01, VUB02 and VUB14 (Mateizel et al. (supra); www.hESCreg.eu) maintained by the applicant, the above taught methods also generated large clusters of Foxa2+ cells, Pdx1+ cells and, Sox9+ cells (Figure 6A, 6B, 6C). Although the extent of Pdx1+ cell differentiation varied between cell lines, in each experiment it was significantly higher when compared to D'Amour *et al.* protocol. Concomitantly, in all these lines, treatment with the present methods led to a significant loss of hepatic markers (AFP and ALB) as described above.

## Claims

1. An *in vitro* method for producing pancreatic progenitor cells or a cell population comprising such, comprising the steps of:
(a) providing cells having definitive endoderm germ layer phenotype; and
(b) exposing the cells of (a) to an antagonist of bone morphogenetic protein (BMP) pathway, an antagonist of hedgehog pathway and an agonist of retinoid signalling.

2. The method according to claim 1, wherein the cells having definitive endoderm germ layer phenotype comprise the expression of any one, more or all of Bry (brachyury), Sox17 (SRY(sex determining region Y)-box 17), Gsc (goosecoid homeobox), Foxa2 (forkhead box A2 ) and CxCR4 (chemokine (C-X-C motif) receptor 4).

3. The method according to any one of claims 1 or 2, wherein said pancreatic progenitor cells comprise the expression of any one, more or all of Pdx1 (pancreatic and duodenal homeobox 1), Foxa2, Sox9 (SRY(sex determining region Y)-box 9) and Hlxb9 (homeo box HB9).

4. The method according to any one of claims 1 to 3, wherein:
- the antagonist of BMP pathway is chosen from the group comprising noggin, chordin, grhemlin, sclerostin, Cerberus, GDF3, Dan, Coco, PRDC, short gastrulation and twisted gastrulation, more preferably the antagonist of BMP pathway is noggin; and/or
- the antagonist of hedgehog pathway is chosen from the group comprising cyclopamine, KAAD-cyclopamine, tomatidine, jervine, cholesterol, tripanarol, AY9944, and anti-hedgehog antibodies, more preferably the antagonist of hedgehog pathway is KAAD-cyclopamine; and/or
- the agonist of retinoid signalling is chosen from the group comprising retinoic acid, retinol and retinal, more preferably wherein said agonist of retinoid signalling is all-trans retinoic acid.

5. The method according to any one of claims 1 to 4, wherein the cells in said step (b) are not exposed to an exogenous agonist of FGF signalling.

6. The method according to any one of claims 1 to 5, wherein the duration of said step (b) is between about 4 days and about 12 days, preferably between about 6 days and about 10 days, more preferably about 7 or 8 days.

7. An *in vitro* method for producing pancreatic endocrine progenitor cells or a cell population comprising such, comprising step (c): exposing the pancreatic progenitor cells or the cell population comprising such, as defined in any one of claims 1 to 6 or as obtainable or directly obtained by the method of any one of claims 1 to 6, to any one or more or all of an agonist of fibroblast growth factor (FGF) signalling, an antagonist of Notch pathway and an agonist of glucagon-like peptide 1 (Glp-1) receptor.

8. The method according to claim 7 comprising the steps (a) and (b) as defined in any one of claims 1 to 6, thereby producing pancreatic progenitor cells or a cell population comprising such, and further comprising step (c): exposing said pancreatic progenitor cells or cell population comprising such to any one, more or all of an agonist of FGF signalling, an antagonist of Notch pathway and an agonist of Glp-1 receptor.

9. The method according to any one of claims 7 or 8, wherein the pancreatic endocrine progenitor cells comprise the expression of any one, more or all of Pdx1, Foxa2, Sox9, Hlxb9, Hnf6 (hepatocyte nuclear factor-6), Nkx6.1 (NK6 homeobox 1) and Ngn3 (neurogenin 3); more preferably comprise the expression of Pdx1, Foxa2, Sox9 and of any one, more or all of Hlxb9, Hnf6, Nkx6.1 and Ngn3; even more preferably comprise at least the expression of Ngn3.

10. The method according to any one of claims 7 to 9, wherein:
- the agonist of FGF signalling is a growth factor selected from the FGF family, more preferably the agonist of FGF signalling is FGF-1 0; and/or
- the antagonist of Notch pathway is chosen from the group comprising gamma secretase/presenilin inhibitors, recombinant notch extracellular domain, activators of notch intracellular domain degradation (SEL-10), and antibodies to notch receptor ligands including delta, serrate, jagged, more preferably the antagonist of Notch pathway is a gamma secretase inhibitor, even more preferably the gamma secretase inhibitor is compound E; and/or
- the agonist of Glp-1 receptor is exendin-4.

11. The method according to any one of claims 7 to 10, wherein the duration of step (c) is between about 1 day and about 8 days, preferably between about 2 days and about 6 days, more preferably between about 3 days and about 5 days, even more preferably about 4 days.

12. The method according to any one of claims 1 to 11, further comprising differentiating *in vitro* the pancreatic progenitor cells or the pancreatic endocrine progenitor cells into pancreatic endocrine cells or a cell population comprising such, more preferably into beta-cells or a cell population comprising such.

13. The method according to any one of claims 1 to 12, wherein the cells are mammalian cells, more preferably human cells.

14. Isolated pancreatic progenitor cells or a cell population comprising such obtainable or directly obtained by the method according to any one of claims 1 to 6 or 13, or isolated pancreatic endocrine progenitor cells or a cell population comprising such obtainable or directly obtained by the method according or any one of claims 7 to 11 or 13, or isolated pancreatic endocrine cells or a cell population comprising such, preferably beta-cells or a cell population comprising such, obtainable or directly obtained by the method according to any one of claims 12 or 13.

15. Isolated pancreatic progenitor cells or a cell population comprising such obtainable or directly obtained by the method according to any one of claims 1 to 6 or 13, or isolated pancreatic endocrine progenitor cells or a cell population comprising such obtainable or directly obtained by the method according or any one of claims 7 to 11 or 13, isolated or pancreatic endocrine cells or a cell population comprising such, preferably beta-cells or a cell population comprising such, obtainable or directly obtained by the method according to any one of claims 12 or 13, for use in therapy, preferably for use in the treatment of conditions or diseases associated with impairment or dysfunction of endocrine pancreas, more preferably for use in the treatment of conditions or diseases associated with impaired beta-cell function, even more preferably for use in the treatment of conditions or diseases associated with impaired glucose homeostasis, for example in the treatment of prediabetes or diabetes.

16. A pharmaceutical composition comprising:
- isolated pancreatic progenitor cells or a cell population comprising such obtainable or directly obtained by the method according to any one of claims 1 to 6 or 13, and/or isolated pancreatic endocrine progenitor cells or a cell population comprising such obtainable or directly obtained by the method according or any one of claims 7 to 11 or 13, and/or isolated pancreatic endocrine cells or a cell population comprising such, preferably beta-cells or a cell population comprising such, obtainable or directly obtained by the method according to any one of claims 12 or 13; and
- one or more pharmaceutically acceptable carriers.
